# EUROPEAN PATENT APPLICATION

(11) **EP 3 643 306 A2**
(43) Date of publication of application: **29.04.2020**
(21) Application number: 19200550.2
(22) Date of filing: 10.08.2012
(51) Int. Cl.: A61K 31/25, A61K 47/60

(54) **CARRIER-LINKED PRODRUGS HAVING REVERSIBLE CARBOXYLIC ESTER LINKAGES**

(30) Priority: 12.08.2011 EP 11177412
(62) Divisional of application: 12745703.4
(71) Applicant: Ascendis Pharma A/S, 2900 Hellerup (DK)
(72) Inventor: Vetter, Dirk, 69120 Heidelberg (DE); Rau, Harald, 69120 Heidelberg (DE)
(74) Representative: Büchel, Edwin

(57) **Abstract**

The invention provides a carrier-linked prodrugs, wherein the biologically active moieties comprise at least one carboxylic acid and wherein the linkage between the drug moiety and linker is in the form of an ester wherein the hydroxyl group required for ester formation is provided by the linker moiety and the carboxyl group required for ester formation is provided by the drug moiety. The hydroxyl group of the linker is sterically hindered by the presence of an alkyl or aryl group on the carbon directly bound to or adjacent to the carbon carrying the hydroxyl group (a-carbon). The steric effect of the alkyl or aryl group enables greater control of the rate of hydrolytic degradation of such carrier-linked prodrugs.

## Description

The present invention is directed to carrier-linked prodrugs having reversible carboxylic ester linkages between carboxyl-comprising biologically active entities and the carrier moiety. Such carrier-linked prodrugs are characterized by slow release of unmodified biologically active entity.

Typically, carriers employed for extended time-action engineering in drug delivery are either used in a non-covalent fashion, with the drug physicochemically formulated into a solvent-carrier mixture, or by permanent covalent attachment of a carrier reagent to one of the drug's functional groups.

Non-covalent drug encapsulation into polymeric carriers has been applied to depot formulations for long-acting release profiles. Typically, the drug is mixed with carrier material and processed in such fashion, that the drug becomes distributed inside the bulk carrier. A disadvantage of the non-covalent approach is that in order to prevent uncontrolled, burst-type release of the drug, encapsulation of the drug has to be highly efficient by creating a sterically highly crowded environment.

Alternatively, the drugs may be conjugated to a carrier through permanent covalent bonds. This approach is applied to various classes of molecules, from so-called small molecules, through natural products up to larger proteins.

Covalent modification of biological molecules with poly(ethylene glycol) has been extensively studied since the late 1970s. So-called PEGylated proteins have shown improved therapeutic efficacy by increasing solubility, reducing immunogenicity, and increasing circulation half-live in vivo due to reduced renal clearance and proteolysis by enzymes (see, for example, Caliceti P.,Veronese F.M., Adv. Drug Deliv. Rev. 2003, 55, 1261-1277).

However, many biological molecules such as IFNalfa2, saquinavir or somatostatin are inactive or show decreased biological activity when a carrier is covalently conjugated to the drug (T. Peleg-Shulman et al., J. Med. Chem., 2004, 47, 4897-4904) and this inactivation is particularly pronounced when natural products or small molecules are used.

In order to avoid shortcomings imposed by either the non-covalent polymer mixtures or the permanent covalent attachment, it may be preferable to employ a prodrug approach for chemical conjugation of the drug to the polymer carrier. In such polymeric prodrugs, the biologically active moieties (drugs, therapeutic, biological molecule, etc.) are typically linked to the polymeric carrier moiety by a temporary bond formed between the carrier moiety and a hydroxyl, amino or carboxy group of the drug molecule.

A number of prodrugs suitable for different functional groups of the drug molecule have been described, for example in WO-A 2005/099768, WO-A 2006/136586, WO-A 2009/095479, und WO-A 2011/012722.

However, only a limited number of prodrug approaches suitable for drugs with carboxyl groups has been described. For example, US7585837B2 discloses prodrugs of carboxyl-comprising biologically active moieties.

However, there exists a need to provide additional approaches for carboxyl-comprising biologically active moieties to allow for a larger range of half-lives and release kinetics.

This object is achieved with a carrier-linked prodrug of formula (Ia) or (Ib):

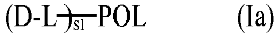

D(̵L-POL)ₛ₂ (Ib),

wherein
each D is individually a biologically active moiety comprising at least one carboxylic acid group;
each POL is individually a carrier moiety which comprises, preferably consists of a polymer with a molecular weight of at least 0.2 kDa,
s1 is an integer from 1 to 64, preferably from 1 to 16, even more preferably s1 is selected from 1, 2, 3, 4, 5, 6, 7 and 8,
s2 is an integer from 1 to 16, preferably from 1 to 8, even more preferably s2 is selected from 1, 2, 3, and 4,
each L is independently a reversible prodrug linker of formula (Ic): 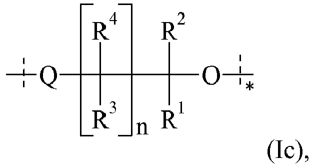 wherein the dashed line marked with an asterisk indicates attachment to a carboxyl group of a biologically active moiety D by forming a carboxylic ester comprising O and the other dashed line indicates attachment to the rest of the molecule;
   R¹ is selected from the group of alkyl, substituted alkyl, aryl, substituted aryl, cycloalkyl and cycloheteroalkyl;
   R² is selected from H, alkyl, and substituted alkyl;
   R³ and R⁴ are independently selected from the group consisting of H, alkyl, and substituted alkyl;
   n is 0 or 1;
   optionally, R¹ and R³ are joined together with the atoms to which they are attached to form a ring A;
   A is selected from the group consisting of C₃₋₁₀ cycloalkyl; 4- to 7-membered aliphatic heterocyclyl; and 9- to 11-membered aliphatic heterobicyclyl;
   Q is a spacer moiety;
or a pharmaceutically acceptable salt thereof.

In a further embodiment, this object is achieved with a carrier-linked prodrug of formula (Id): 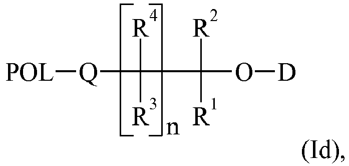
wherein D is linked to the rest of the molecule through a carboxyl group of D by forming a carboxylic ester comprising O;
R¹ is selected from the group of alkyl, substituted alkyl, aryl, substituted aryl, cycloalkyl and cycloheteroalkyl;
R² is selected from H, alkyl, and substituted alkyl;
R³ and R⁴ are independently selected from the group consisting of H, alkyl, and substituted alkyl;
n is 0 or 1;
optionally, R¹ and R³ are joined together with the atoms to which they are attached to form a ring A;
A is selected from the group consisting of C₃₋₁₀ cycloalkyl; 4- to 7-membered aliphatic heterocyclyl; and 9- to 11-membered aliphatic heterobicyclyl;
Q is a spacer moiety;
D is a biologically active moiety comprising at least one carboxylic acid group;
POL is a carrier moiety which comprises, preferably consists of a polymer with a molecular weight of at least 0.2 kDa,
or a pharmaceutically acceptable salt thereof.

It was surprisingly found that prodrugs of formula (Ia) and (Ib) exhibit therapeutically useful autohydrolysis (autocatalytic cleavage) and that the steric effect of the alkyl or aryl group of R¹ enables greater control of the rate of hydrolytic degradation of such carrier linked prodrugs.

The presence of the moiety R¹ confers greater stability to hydrolysis of the carboxylic ester linkage due to the steric and electronic effect of the alkyl or aryl group. The steric effect may be increased by increasing the size of the alkyl or aryl group, as would be the case in replacing methyl with ethyl.

The structure used as the R¹ moiety, the half-lives of the prodrugs of the invention can be controlled and manipulated. Better control of the rate of hydrolytic degradation enables the practitioner to tailor constructs for specific end uses that require certain degradation properties.

Within the present invention the terms are used having the meaning as follows.

The terms "drug", "biologically active molecule", "biologically active moiety", "biologically active agent", "active agent", "active substance" and the like mean any substance which can affect any physical or biochemical properties of a biological organism, including but not limited to viruses, bacteria, fungi, plants, animals, and humans. In particular, as used herein, the terms include any substance intended for diagnosis, cure, mitigation, treatment, or prevention of disease in organisms, in particular humans or animals, or to otherwise enhance physical or mental well-being of organisms, in particular humans or animals.

"Biologically active moiety D" or "carboxyl-comprising biologically active moiety D" means the part of the biologically active moiety-reversible prodrug linker conjugate or the part of the biologically active moiety-reversible prodrug linker-carrier conjugate, which results after cleavage in a drug D-OH of known biological activity, wherein -OH is part of the carboxyl group.

Targeting moieties are moieties that when present in a molecule, such as for example a prodrug, allow preferential localization of such larger molecule in specific target areas of the organism to which it has been administered. Such specific target areas might be organs, certain cell types or subcellular compartments. "Preferential localization" means that at least 10%, preferably at least 20% and more preferably at least 30% of the biologically active moieties administered to a patient reach said specific target areas.

Targeting moieties may be divided into 3 classes according to size:
- small molecular targeting moieties, for example C-glucuronide, cobalamin, vitamins such as folic acid (folate) and analogs and derivatives, carbohydrates, bisphosphonates, N-acetylgalactosamine,
- peptides, for example bombesin, somatostatin, LHRH, EGF, VEGF, hCG, fragments of luteinizing hormone (LH), octreotide, vapreotide, lanreotide, RC-3940 series, decapeptyl, lupron, zoladex, cetrorelix, peptides or peptidomimetics containing the NGR or RGD motifs or derived from these motifs such as CNGRC (linear), GNGRG (cyclic), ACDC RGD CFCG (cyclic), CDCRGDCFC, CNGRC (cyclic), CRGDCGG, CNGRC, or other peptides such as ATWLPPR, thrombospondin (TSP)-1 mimetics, (RGD peptidomimetic), CTTHWGFTLC, CGNKRTRGC, neuropeptide substance P, SSP, the Sar9, Met(O2)11 analog of substance P, cholecystokinin (CCK), corticotropin-releasing hormone/factor (CRH/CRF), dermorphin, FGF-2 or basic fibroblast growth factor, galanin, melanopsin, neurotensin,
- and protein or macro- molecular targeting moieties, for example IL-2, GM-CSF, TNF-a, transferrin, immunoglobulins, acetylated-LDL, lactoferrin (Lf) (also called lactotransferrin) and lactoferricin (Lcin), gambogic acid (GA), antibody fragments and affinity scaffold proteins.

In principle, any ligand of a cell surface receptor may be advantageously used as a targeting moiety. For instance, ATWLPPR peptide is a potent antagonist of VEGF; thrombospondin-1 (TSP-1) induces apoptosis in endothelial cells, RGD-motif mimics block integrin receptors, NGR-containing peptides inhibit aminopeptidase N, and cyclic peptides containing the sequence of HWGF selectively inhibit MMP-2 and MMP-9. LyP-1 peptide specifically binds to tumor lymphatic vessels. Illustrative other ligands include peptide ligands identified from library screens, tumor cell-specific peptides, tumor cell-specific aptamers, tumor cell-specific carbohydrates, tumor cell-specific monoclonal or polyclonal antibodies, Fab or scFv (i.e., a single chain variable region) fragments of antibodies such as, for example, a Fab fragment of an antibody directed to EphA2 or other proteins specifically expressed or uniquely accessible on metastatic cancer cells, small organic molecules derived from combinatorial libraries, growth factors, such as EGF, FGF, insulin, and insulin-like growth factors, and homologous polypeptides, somatostatin and its analogs, transferrin, lipoprotein complexes, bile salts, selecting, steroid hormones, Arg-Gly-Asp containing peptides, retinoids, various Galectins, δ-opioid receptor ligands, cholecystokinin A receptor ligands, ligands specific for angiotensin AT1 or AT2 receptors, peroxisome proliferator-activated receptor λ ligands, β-lactam antibiotics such as penicillin, small organic molecules including antimicrobial drugs, and other molecules that bind specifically to a receptor preferentially expressed on the surface of tumor cells or on an infectious organism, antimicrobial and other drugs designed to fit into the binding pocket of a particular receptor based on the crystal structure of the receptor or other cell surface protein, ligands of tumor antigens or other molecules preferentially expressed on the surface of tumor cells,or fragments of any of these molecules. Examples of tumor-specific antigens that can function as targeting moieties include extracellular epitopes of a member of the ephrin family of proteins, such as EphA2. EphA2 expression is restricted to cell-cell junctions in normal cells, but EpbA2 is distributed over the entire cell surface in metastatic tumor cells. Thus, EphA2 on metastatic cells would be accessible for binding to, for example, a Fab fragment of an antibody conjugated to an immunogen, whereas the protein would not be accessible for binding to the Fab fragment on normal cells, resulting in a targeting moiety specific for metastatic cancer cells.

Further examples for such targeting moieties are: FSH-33, allatostatin 1, hepatocarcinoma targeting peptide, peptide GFE, anti-EGFR antibodies and/or antibody fragments, in particular cetuximab, CendR, iRGD peptide (RGD-CendR hybrid peptide), small molecules, antibodies and/or antibody fragments binding to cancer-specific epitopes like e.g. CEA, gastrin-releasing peptide receptors, somatostatin receptors, galanin receptors, follicle-stimulating hormone receptors, p32 protein, fibroblast growth factor receptors, HepG2, epidermal growth factor receptors, integrin αvβ6, neuropilin-1 receptor and VEGF receptors.

"Free form" of a drug refers to the drug in its unmodified, pharmacologically active form, such as after being released from a carrier-linked prodrug.

To enhance physicochemical or pharmacokinetic properties of a drug *in vivo,* such drug can be conjugated with a carrier, as in the present invention. If the drug is reversibly bound to a carrier and a linker, as in the present invention, such system is commonly assigned as "carrier-linked prodrug". According to the definitions provided by IUPAC (as given under http://www.chem.qmul.ac.uk/iupac/medchem/ah.html, accessed on March 7, 2011), a carrier-linked prodrug is a prodrug that contains a temporary, or reversible, linkage of a given active substance with a reversible carrier group that produces improved physicochemical or pharmacokinetic properties and that can be easily removed *in vivo,* usually by a hydrolytic cleavage.

The term "promoiety" refers to the part of the prodrug which is not the drug, thus meaning for example the carrier moiety/moieties POL, as well as the reversible prodrug linker moiety/moieties, respectively.

The term "hyperbranched moiety" or "branched moiety" refers to a moiety comprising at least one branching point. Such branching point comprises, for example, an at least 3-fold substituted carbocycle, an at least 3-fold substituted heterocycle, a tertiary carbon atom, a quaternary carbon atom or a tertiary nitrogen atom.

The term "branch" refers to those moieties of a branched spacer moiety that connect branching points or to those moieties that are terminally connected to branching points.

The term "reversible prodrug linker" or "transient prodrug linkers" refers to a moiety which on its one end is attached to the biologically active moiety through a reversible linkage and at another end is permanently attached to the moiety POL. Such reversible prodrug linkers are non-enzymatically hydrolytically degradable, i.e. cleavable, under physiological conditions (aqueous buffer at pH 7.4, 37°C) with half-lives ranging from, for example, one hour to three months. In the carrier-linked prodrugs of the present invention, the reversible linkage is a carboxylic ester.

Permanent linkages are non-enzymatically hydrolytically degradable under physiological conditions (aqueous buffer at pH 7.4, 37°C) with half-lives of six months or longer, such as, for example, amides.

The phrases "in bound form", "connected to" or "moiety" refer to sub-structures which are part of a molecule. The phrases "in bound form" and "connected to" are used to simplify reference to moieties by naming or listing reagents, starting materials or hypothetical starting materials well known in the art, and whereby "in bound form" and "connected to" mean that for example one or more hydrogen radicals (-H), or one or more activating or protecting groups present in the reagents or starting materials are not present in the moiety when part of a molecule.

The term "polymer" describes a molecule comprising, in particular consisting of repeating structural units connected by chemical bonds in a linear, circular, branched, crosslinked or dendrimeric way or a combination thereof, which can be of synthetic or biological origin or a combination of both. It is understood, that e.g. capping moieties may be present in a polymer.

The term "polymeric" refers to a moiety comprising one or more polymer(s).

The term "poly(ethylene glycol)-based polymeric chain" or "PEG-based polymeric chain" refers to a polymer comprising at least 20 weight % ethylene glycol moieties, more preferably at least 50% by weight, even more preferably at least 80% by weight ethylene glycol moieties, which chain is optionally capped and/or optionally further comprises one or more functional groups, for example amine group(s). Such one or more functional groups allow the covalent connection to a moiety Q of the reversible linker moiety L. It is understood that a PEG-based polymeric chain may be terminated or interrupted by alkyl or aryl groups and optionally be substituted with heteroatoms and/or functional groups. Suitable capping or terminating groups for a PEG-based polymeric chain are for example CH₃- , CH₃-O- and CH₃-CH₂-.

A "peptide" is a single linear polymer chain of up to about 100 amino acids, preferably up to about 50 amino acids, more preferably up to about 25 amino acids bonded together by peptide bonds between the carboxyl and amino groups of adjacent amino acid residues. Preferably, a peptide is a single linear polymer chain of at least about 4 amino acids, more preferably of at least about 6 amino acids. A "protein" or "polypeptide" is a single linear polymer chain of more than about 100 amino acids bonded together by peptide bonds between the carboxyl and amino groups of adjacent amino acid residues. Proteins or polypeptides may comprise modifications, for example by C-terminal amidation.

The term "peptide fragment" as used herein refers to a polypeptide moiety or peptide moiety comprising at least 3 amino acids and comprising at least one alanine, and/or one serine and/or one proline.

The term "polymer cassette" relates to peptides of defined, individual amino acid stretches. Polymer cassettes may be used to form a protein carrier POL. Thus, a protein carrier POL comprises, preferably consists of one or more, in particular of 1, 2, 3, 4, 5, 6, 7, 8, 9, 10, 11, 12, 13, 14, 15, 16, 17, 18, 19 or 20 polymer cassette(s), which may be of the same or of different sequence.

As used herein, the term "random coil" relates to any conformation of a polymeric molecule, including proteins, in which the individual monomeric elements that form said polymeric structure are essentially randomly oriented towards the adjacent monomeric elements while still being chemically bound to said adjacent monomeric elements. In particular, a polypeptide or protein having random coil conformation substantially lacks a defined secondary and tertiary structure. The nature of polypeptide random coils and their methods of experimental identification are known to the person skilled in the art. In particular, the lack of secondary and tertiary structure of a protein may be determined by circular dichroism (CD) measurements. CD spectroscopy represents a light absorption spectroscopy method in which the difference in absorbance of right- and left-circularly polarized light by a substance is measured. The secondary structure of a protein can be determined by CD spectroscopy using far-ultraviolet spectra with a wavelength between approximately 190 and 250 nm. At these wavelengths the different secondary structures commonly found in conformations each give rise to a characteristic shape and magnitude of the CD spectrum. Accordingly, by using CD spectrometry the skilled artisan is readily capable of determining whether an amino acid polymer adopts random coil conformation at physiological conditions.

When determining whether a peptide or protein adopts random coil conformation under experimental conditions using the methods as described herein, the biophysical parameters such as temperature, pH, osmolarity and protein content may be different to the physiological conditions normally found in vivo. Temperatures between 1 °C and 42 °C or preferably 4 °C to 25 °C may be considered useful to test and/or verify the biophysical properties and biological activity of a peptide or protein under physiological conditions in vitro.

Several buffers, in particular in experimental settings (for example in the determination of protein structures, in particular in circular dichroism (CD) measurements and other methods that allow the person skilled in the art to determine the structural properties of a protein/polypeptide or peptide stretch) or in buffers, solvents and/or excipients for pharmaceutical compositions, are considered to represent "physiological solutions" or "physiological conditions" in vitro. Examples of such buffers are, e.g. phosphate-buffered saline (PBS: 115 mM NaCl, 4 mM KH₂PO₄, 16 mM Na₂HPO₄ pH 7.4), Tris buffers, acetate buffers, citrate buffers or similar buffers such as those used in the appended examples. Generally, the pH of a buffer representing physiological conditions should lie in a range from 6.5 to 8.5, preferably in a range from 7.0 to 8.0, most preferably in a range from 7.2 to 7.7 and the osmolarity should lie in a range from 10 to 1000 mmol/kg H₂O, more preferably in a range from 50 to 500 mmol/kg H₂O and most preferably in a range from 200 to 350 mmol/kg H₂O. Optionally, the protein content of a buffer representing physiological conditions may lie in a range from 0 to 100 g/l, neglecting the protein with biological activity itself, whereby typical stabilizing proteins may be used, for example human or bovine serum albumin.

Other established biophysical methods for determining random coil conformation include nuclear magnetic resonance (NMR) spectroscopy, absorption spectrometry, infrared and Raman spectroscopy, measurement of the hydrodynamic volume via size exclusion chromatography, analytical ultracentrifugation and dynamic/static light scattering as well as measurements of the frictional coefficient or intrinsic viscosity.

The term "hydrogel" refers to a three-dimensional, hydrophilic or amphiphilic polymeric network capable of taking up large quantities of water. Such network may be composed of homopolymers or copolymers, and is insoluble due to the presence of covalent chemical or physical (ionic, hydrophobic interactions, entanglements) crosslinks. The crosslinks provide the network structure and physical integrity. Hydrogels exhibit a thermodynamic compatibility with water which allows them to swell in aqueous media. The chains of the network are connected in such a fashion that pores exist and that a substantial fraction of these pores are of dimensions between 1 nm and 1000 nm.

The term "water soluble" refers to a molecule that is soluble in water at room temperature. Typically, a solution of a water-soluble molecule will transmit at least about 75%, more preferably at least about 95% of light, transmitted by the same solution after filtering. On a weight basis, a water-soluble molecule or parts thereof will preferably be at least about 35% (by weight) soluble in water, more preferably at least about 50% (by weight) soluble in water, still more preferably about 70% (by weight) soluble in water, and still more preferably about 85% (by weight) soluble in water. It is most preferred, however, that the water-soluble molecule or parts thereof is about 95% (by weight) soluble in water or completely soluble in water.

The term "functional group" refers to specific groups of atoms within molecules that can undergo characteristic chemical reactions. Examples of functional groups are hydroxyl, carbonyl, aldehyde, carboxyl, ester, ketal, hemiketal, acetal, hemiacetal, primary/secondary/tertiary amine, cyanate, disulfide, sulfhydryl, sulfonyl, phosphate.

If a functional group is coupled to another functional group, the resulting chemical structure is referred to as "linkage". For example, the reaction of an amine functional group with a carboxyl functional group results in an amide linkage. Further examples for linkages are ester, ether, ketal, acetal, secondary/tertiary amine, carboxamide, sulfide, and disulfide.

"Alkyl" means a straight-chain (linear, unbranched) or branched carbon chain (unsubstituted alkyl). Optionally, one or more hydrogen atom(s) of an alkyl carbon may be replaced by a substituent as indicated herein. In general, a preferred alkyl is C₁₋₆ alkyl.

"C₁₋₄ alkyl" means an alkyl chain having 1 to 4 carbon atoms (unsubstituted C₁₋₄ alkyl), e.g. if present at the end of a molecule: methyl, ethyl, n-propyl, isopropyl, n-butyl, isobutyl, sec-butyl tert-butyl, or e.g. -CH₂-, -CH₂-CH₂-, -CH(CH₃)-, -CH₂-CH₂-CH₂-, -CH(C₂H₅)-, - C(CH₃)₂-, when two moieties of a molecule are linked by the alkyl group (also referred to as C₁₋₄ alkylene). Optionally, one or more hydrogen atom(s) of a C₁₋₄ alkyl carbon may be replaced by a substituent as indicated herein. Accordingly, "C₁₋₅₀ alkyl" means an alkyl chain having 1 to 50 carbon atoms.

"C₁₋₆ alkyl" means an alkyl chain having 1 - 6 carbon atoms, e.g. if present at the end of a molecule: C₁₋₄ alkyl, methyl, ethyl, n-propyl, isopropyl, n-butyl, isobutyl, sec-butyl, tert-butyl, n-pentyl, n-hexyl, or e.g. -CH₂-, -CH₂-CH₂-, -CH(CH₃)-, -C(CH₂)-, -CH₂-CH₂-CH₂-, - CH(C₂H₅)-, -C(CH₃)₂-, when two moieties of a molecule are linked by the alkyl group (also referred to as C₁₋₆ alkylene). One or more hydrogen atom(s) of a C₁₋₆ alkyl carbon may be replaced by a substituent as indicated herein. The terms C₁₋₁₅ alkyl or C₁₋₁₅ alkylene are defined accordingly.

"C₂₋₆ alkenyl" means an alkenyl chain having 2 to 6 carbon atoms, e.g. if present at the end of a molecule: -CH=CH₂, -CH=CH-CH₃, -CH₂-CH=CH₂, -CH=CH-CH₂-CH₃, -CH=CH-CH=CH₂, or e.g. -CH=CH-, when two moieties of a molecule are linked by the alkenyl group. One or more hydrogen atom(s) of a C₂₋₆ alkenyl carbon may be replaced by a substituent as indicated herein.

The term C₂₋₄ alkenyl is defined accordingly.

"C₂₋₆ alkynyl" means an alkynyl chain having 2 to 6 carbon atoms, e.g. if present at the end of a molecule: -C≡CH, -CH₂-C≡CH, CH₂-CH₂-C≡CH, CH₂-C≡C-CH₃, or e.g. -C≡C- when two moieties of a molecule are linked by the alkynyl group. One or more hydrogen atom(s) of a C₂₋₆ alkynyl carbon may be replaced by a substituent as indicated herein. The term C₂₋₄ alkynyl is defined accordingly.

"C₂₋₅₀ alkenyl" means a branched or unbranched alkenyl chain having 2 to 50 carbon atoms (unsubstituted C₂₋₅₀ alkenyl), e.g. if present at the end of a molecule: -CH=CH₂, -CH=CH-CH₃, -CH₂-CH=CH₂, -CH=CH-CH₂-CH₃, -CH=CH-CH=CH₂, or e.g. -CH=CH-, when two moieties of a molecule are linked by the alkenyl group. Optionally, one or more hydrogen atom(s) of a C₂₋₅₀ alkenyl carbon may be replaced by a substituent as further specified. Accordingly, the term "alkenyl" relates to a carbon chain with at least one carbon carbon double bond. Optionally, one or more triple bonds may occur. The term "C₂₋₁₅ alkenyl" is defined accordingly.

"C₂₋₅₀ alkynyl" means a branched or unbranched alkynyl chain having 2 to 50 carbon atoms (unsubstituted C₂₋₅₀ alkynyl), e.g. if present at the end of a molecule: -C≡CH, -CH₂-C≡CH, CH₂-CH₂-C≡CH, CH₂-C≡C-CH₃, or e.g. -C≡C- when two moieties of a molecule are linked by the alkynyl group. Optionally, one or more hydrogen atom(s) of a C₂₋₅₀ alkynyl carbon may be replaced by a substituent as further specified. Accordingly, the term "alkynyl" relates to a carbon chain with at least one carbon triple bond. Optionally, one or more double bonds may occur.

"C₃₋₇ cycloalkyl" or "C₃₋₇ cycloalkyl ring" means a cyclic alkyl chain having 3 to 7 carbon atoms, which may have carbon-carbon double bonds being at least partially saturated (unsubstituted C₃₋₇ cycloalkyl), e.g. cyclopropyl, cyclobutyl, cyclopentyl, cyclohexyl, cyclohexenyl, cycloheptyl. Optionally, one or more hydrogen atom(s) of a cycloalkyl carbon may be replaced by a substituent as indicated herein. The term "C₃₋₇ cycloalkyl" or "C₃₋₇ cycloalkyl ring" also includes bridged bicycles like norbonane (norbonanyl) or norbonene (norbonenyl). Accordingly, "C₃₋₅ cycloalkyl" means a cycloalkyl having 3 to 5 carbon atoms. Accordingly, "C₃₋₁₀ cycloalkyl" means a cycloalkyl having 3 to 10 carbon atoms.

"Halogen" means fluoro, chloro, bromo or iodo. It is generally preferred that halogen is fluoro or chloro.

"4 to 7 membered heterocyclyl" or "4 to 7 membered heterocycle" means a ring with 4, 5, 6 or 7 ring atoms that may contain up to the maximum number of double bonds (aromatic or non-aromatic ring which is fully, partially or un-saturated) wherein at least one ring atom up to 4 ring atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom (unsubstituted 4 to 7 membered heterocyclyl). For the sake of completeness it is indicated that in some embodiments of the present invention, 4 to 7 membered heterocyclyl has to fulfill additional requirements. Examples for a 4 to 7 membered heterocycles are azetidine, oxetane, thietane, furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, diazepane, azepine or homopiperazine. Optionally, one or more hydrogen atom(s) of a 4 to 7 membered heterocyclyl may be replaced by a substituent.

"8 to 11 membered heterobicyclyl" or "8 to 11 membered heterobicycle" means a heterocyclic system of two rings with 8 to 11 ring atoms, where at least one ring atom is shared by both rings and that may contain up to the maximum number of double bonds (aromatic or non-aromatic ring which is fully, partially or un-saturated) wherein at least one ring atom up to 6 ring atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom (unsubstituted 8 to 11 membered heterobicyclyl). Examples for a 8 to 11 membered heterobicycle are indole, indoline, benzofuran, benzothiophene, benzoxazole, benzisoxazole, benzothiazole, benzisothiazole, benzimidazole, benzimidazoline, quinoline, quinazoline, dihydroquinazoline, quinoline, dihydroquinoline, tetrahydroquinoline, decahydroquinoline, isoquinoline, decahydroisoquinoline, tetrahydroisoquinoline, dihydroisoquinoline, benzazepine, purine or pteridine. The term 8 to 11 membered heterobicycle also includes spiro structures of two rings like 1,4-dioxa-8-azaspiro[4.5]decane or bridged heterocycles like 8-aza-bicyclo[3.2.1]octane. The term "9 to 11 membered heterobicyclyl" or "9 to 11 membered heterobicycle" is defined accordingly.

The term "aliphatic" means fully saturated.

The term "interrupted" means that between two carbon atoms of, for example, a linker or a spacer or at the respective end of the carbon chain between the respective carbon atom and the hydrogen atom a group (such a -O- or -NH-) is inserted.

In general the term "substituted" preferably refers to substituents, which are the same or different and which are independently selected from the group consisting of halogen, CN, COOR^{b9}, OR^{b9}, C(O)R^{b9}, C(O)N(R^{b9}R^{b9a}), S(O)₂N(R^{b9}R^{b9a}), S(O)N(R^{b9}R^{b9a}), S(O)₂R^{b9}, S(O)R^{b9}, N(R^{b9})S(O)₂N(R^{b9a}R^{b9b}), SR^{b9}, N(R^{b9}R^{b9a}), NO₂, OC(O)R^{b9}, N(R^{b9})C(O)R^{b9a}, N(R^{b9})S(O)₂R^{b9a}, N(R^{b9})S(O)R^{b9a}, N(R^{b9})C(O)OR^{b9a}, N(R^{b9})C(O)N(R^{b9a}R^{b9b}), OC(O)N(R^{b9}R^{b9a}), T^{b}, C₁₋₅₀ alkyl, C₂₋₅₀ alkenyl, and C₂₋₅₀ alkynyl,
wherein T^{b}, C₁₋₅₀ alkyl, C₂₋₅₀ alkenyl, and C₂₋₅₀ alkynyl are optionally substituted with one or more R^{b10}, which are the same or different, and wherein C₁₋₅₀ alkyl; C₂₋₅₀ alkenyl; and C₂₋₅₀ alkynyl are optionally interrupted by one or more groups selected from the group consisting of T^{b}, -C(O)O-; -O-; -C(O)-; -C(O)N(R^{b11})-; _S(O)₂N(R^{b11})-, -S(O)N(R^{b11})-; -S(O)₂-; -S(O)-; -N(R^{b11})S(O)₂N(R^{b11a})-; -S-; -N(R^{b11})-; -OC(O)R^{b11}; -N(R^{b11})C(O)-; -N(R^{b11})S(O)₂-; -N(R^{b11})S(O)-; -N(R^{b11})C(O)O-; -N(R^{b11})C(O)N(R^{b11a})-; and -OC(O)N(R^{b11}R^{b11a});
R^{b9}, R^{b9a}, R^{b9b} are independently selected from the group consisting of H; T^{b}; and C₁₋₅₀ alkyl; C₂₋₅₀ alkenyl; and C₂₋₅₀ alkynyl,
   wherein T^{b}, C₁₋₅₀ alkyl, C₂₋₅₀ alkenyl, and C₂₋₅₀ alkynyl are optionally substituted with one or more R^{b10}, which are the same or different, and wherein C₁₋₅₀ alkyl; C₂₋₅₀ alkenyl; and C₂₋₅₀ alkynyl are optionally interrupted by one or more groups selected from the group consisting of T^{b}, -C(O)O-, -O-, -C(O)-, -C(O)N(R^{b11})-, - S(O)₂N(R^{b11})-, -S(O)N(R^{b11})-, -S(O)₂-, -S(O)-, -N(R^{b11})S(O)₂N(R^{b11a})-, -S-, - N(R^{b11})-, -OC(O)R^{b11}, -N(R^{b11})C(O)-, -N(R^{b11})S(O)₂-, -N(R^{b11})S(O)-, - N(R^{b11})C(O)O-, -N(R^{b11})C(O)N(R^{b11a})-, and -OC(O)N(R^{b11}R^{b11a}),
   T^{b} is selected from the group consisting of phenyl, naphthyl, indenyl, indanyl, tetralinyl, C₃₋₁₀ cycloalkyl, 4- to 7-membered heterocyclyl, and 9- to 11-membered heterobicyclyl, wherein T^{b} is optionally substituted with one or more R^{b10}, which are the same or different,
   R^{b10} is halogen, CN, oxo (=O), COOR^{b12}, OR^{b12}, C(O)R^{b12}, C(O)N(R^{b12}R^{b12a}), S(O)₂N(R^{b12}R^{b12a}), S(O)N(R^{b12}R^{b12a}), S(O)₂R^{b12}, S(O)R^{b12}, N(R^{b12})S(O)₂N(R^{b12a}R^{b12b}), SR^{b12}, N(R^{b12}R^{b12a}), NO₂, OC(O)R^{b12}, N(R^{b12})C(O)R^{b12a}, N(R^{b12})S(O)₂R^{b12a}, N(R^{b12})S(O)R^{b12a}, N(R^{b12})C(O)OR^{b12a}, N(R^{b12})C(O)N(R^{b12a}R^{b12b}), OC(O)N(R^{b12}R^{b12a}), or C₁₋₆ alkyl, wherein C₁₋₆ alkyl is optionally substituted with one or more halogen, which are the same or different,
   R^{b11}, R^{b11a}, R^{b12}, R^{b12a}, R^{b12b} are independently selected from the group consisting of H; or C₁₋₆ alkyl, wherein C₁₋₆ alkyl is optionally substituted with one or more halogen, which are the same or different.

The term "interrupted" means that between two carbons a group is inserted or that at the end of the carbon chain between the carbon and hydrogen.

In general the term "comprise" or "comprising" also encompasses "consist of' or "consisting of".

The terms "spacer", "spacer group", "spacer molecule", and "spacer moiety" are used interchangeably and refer to any moiety suitable for connecting two moieties, such as C₁₋₅₀ alkyl, C₂₋₅₀ alkenyl or C₂₋₅₀ alkinyl, which fragment is optionally interrupted by one or more groups selected from -NH-, -N(C₁₋₄ alkyl)-, -O-, -S-, -C(O)-, -C(O)NH-, -C(O)N(C₁₋₄ alkyl)-, - O-C(O)-, -S(O)-, -S(O)₂-, 4- to 7-membered heterocyclyl, phenyl or naphthyl.

The term "terminus" refers to the last carbon atom or heteroatom of a linear or branched chain of carbon atoms and/or heteroatoms, i.e. "terminus" refers to a carbon or heteroatom which is connected to exactly one other carbon or heteroatom.

The term "interrupted" means that between two carbon atoms of, for example, a linker or a spacer or at the respective end of the carbon chain between the respective carbon atom and the hydrogen atom a group (such a -O- or -NH-) is inserted.

The present invention relates to a carrier-linked prodrug of formula (Ia) or (Ib):

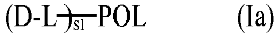

D(̵L-POL)ₛ₂ (Ib),

wherein
each D is independently a biologically active moiety comprising at least one carboxylic acid group;
each POL is independently a carrier moiety which comprises, preferably consists of a polymer with a molecular weight of at least 0.2 kDa,
s1 is an integer ranging from 1 to 64, preferably ranging from 1 to 16, even more preferably s1 is selected from 1, 2, 3, 4, 5, 6, 7 and 8,
s2 is an integer ranging from 1 to 16, preferably ranging from 1 to 8, even more preferably s2 is selected from 1, 2, 3, and 4,
each L is independently a reversible prodrug linker of formula (Ic): 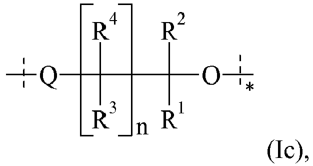
   wherein the dashed line marked with an asterisk indicates attachment to the carboxyl group of a biologically active moiety D by forming a carboxylic ester comprising O and the other dashed line indicates attachment to the rest of the molecule;
   R¹ is selected from the group of unsubstituted alkyl; substituted alkyl; unsubstituted phenyl; substituted phenyl; unsubstituted naphthyl; substituted naphthyl; unsubstituted indenyl; substituted indenyl; unsubstituted indanyl; substituted indanyl; unsubstituted tetralinyl; substituted tetralinyl; unsubstituted C₃₋₁₀ cycloalkyl; substituted C₃₋₁₀ cycloalkyl; unsubstituted 4- to 7-membered heterocyclyl; substituted 4- to 7-membered heterocyclyl; unsubstituted 9- to 11-membered heterobicyclyl; and substituted 9- to 11-membered heterobicyclyl;
   R² is selected from H, unsubstituted alkyl, and substituted alkyl;
   R³ and R⁴ are independently selected from the group consisting of H, unsubstituted alkyl, and substituted alkyl;
   n is 0 or 1;
   optionally, R¹ and R³ are joined together with the atoms to which they are attached to form a ring A;
   A is selected from the group consisting of C₃₋₁₀ cycloalkyl; 4- to 7-membered aliphatic heterocyclyl; and 9- to 11-membered aliphatic heterobicyclyl, wherein A is unsubstituted or substituted;
   Q is a spacer moiety;
or a pharmaceutically acceptable salt thereof.

Accordingly, a carrier-linked prodrug of the present invention has the formula (IA) or (IB): 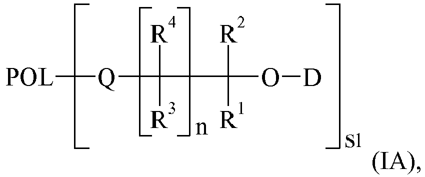 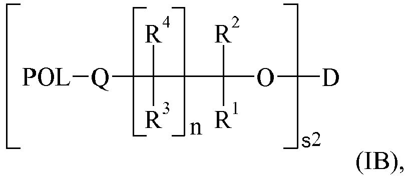 wherein R1, R2, R3, R4, Q, POL and D are used as defined in formula (Ia) and (Ib).

In a preferred embodiment, a carrier-linked prodrug has the structure of formula (Ia). In such embodiment, s1 sub-structures D-L 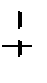 are connected to POL. In a preferred embodiment, the moiety POL comprises at least s1 functional groups, which are connected to the s1 sub-structures D-L 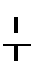 .

In such carrier-linked prodrugs of formula (Ia) each moiety D and each moiety L may be the same or different. Preferably, all moieties L and all moieties D of the carrier-linked produg of formula (Ia) are the same.

In another preferred embodiment, s1 of formula (Ia) is 1.

In a further preferred embodiment, s1 of formula (Ia) is 2.

In a further preferred embodiment, a carrier-linked prodrug has the structure of formula (Ib). In such embodiment, s2 of formula (Ib) sub-structures POL-L 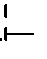 are connected to D. In such preferred embodiment, the moiety D comprises at least s2 carboxylic acid groups, which are connected to the s2 sub-structures POL-L 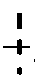 .

In case s2 of formula (Ib) is at least 2, the s2 moieties L may be the same or different, preferably are the same.

In case s2 of formula (Ib) is at least 2, the s2 moieties POL in a carrier-linked prodrug of the present invention may be the same or different, preferably are the same.

In another preferred embodiment, s2 of formula (Ib) is 1.

In a further preferred embodiment, s2 of formula (Ib) is 2.

In a further embodiment, the present invention relates to a carrier-linked prodrug of formula (Id): 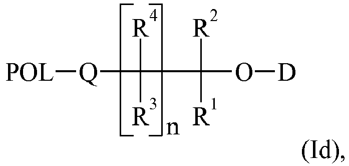
wherein D is linked to the rest of the molecule through a carboxyl group of D by forming a carboxylic ester comprising O;
R¹ is selected from the group of unsubstituted alkyl; substituted alkyl; unsubstituted phenyl; substituted phenyl; unsubstituted naphthyl; substituted naphthyl; unsubstituted indenyl; substituted indenyl; unsubstituted indanyl; substituted indanyl; unsubstituted tetralinyl; substituted tetralinyl; unsubstituted C₃₋₁₀ cycloalkyl; substituted C₃₋₁₀ cycloalkyl; unsubstituted 4- to 7-membered heterocyclyl; substituted 4- to 7-membered heterocyclyl; unsubstituted 9- to 11-membered heterobicyclyl; and substituted 9- to 11-membered heterobicyclyl;
R² is selected from H, unsubstituted alkyl, and substituted alkyl;
R³ and R⁴ are independently selected from the group consisting of H, unsubstituted alkyl, and substituted alkyl;
n is 0 or 1;
optionally, R¹ and R³ are joined together with the atoms to which they are attached to form a ring A;
A is selected from the group consisting of C₃₋₁₀ cycloalkyl; 4- to 7-membered aliphatic heterocyclyl; and 9- to 11-membered aliphatic heterobicyclyl, wherein A is unsubstituted or substituted;
Q is a spacer moiety;
D is a biologically active moiety comprising at least one carboxylic acid group;
POL is a carrier moiety which comprises, preferably consists of a polymer with a molecular weight of at least 0.2 kDa,
or a pharmaceutically acceptable salt thereof.

The moiety of formula (Ic) 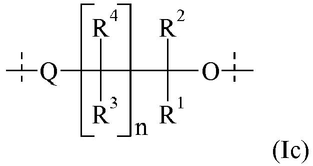 represents the moiety L of formulas (Ia) and (Ib), wherein Q, R¹, R², R³ and R⁴ are used as defined in formula (Id).

Preferably, R¹ of formula (Ic) or (Id) is C₁₋₆ alkyl or substituted C₁₋₆ alkyl, more preferably C₁₋₄ alkyl or substituted C₁₋₄ alkyl.

More preferably, R¹ of formula (Ic) or (Id) is selected from methyl, ethyl, n-propyl, isopropyl, n-butyl, isobutyl, sec-butyl, t-butyl, and benzyl.

Preferably, R² of formula (Ic) or (Id) is H.

Preferably, R³ of formula (Ic) or (Id) is H, C₁₋₆ alkyl or substituted C₁₋₆ alkyl, more preferably C₁₋₄ alkyl or substituted C₁₋₄ alkyl. More preferably, R³ is selected from methyl, ethyl, n-propyl, isopropyl, n-butyl, isobutyl, sec-butyl, t-butyl, and benzyl.

More preferably, R³ of formula (Ic) or (Id) is H.

Preferably, R⁴ of formula (Ic) or (Id) is s H, C₁₋₆ alkyl or substituted C₁₋₆ alkyl, more preferably C₁₋₄ alkyl or substituted C₁₋₄ alkyl. More preferably, R⁴ is selected from methyl, ethyl, n-propyl, isopropyl, n-butyl, isobutyl, sec-butyl, t-butyl, and benzyl.

More preferably, R⁴ of formula (Ic) or (Id) is H.

In another preferred embodiment, R¹ and R³ of formula (Ic) or (Id) are joined together with the atoms to which they are attached to form a ring A; wherein A is selected from the group consisting of cyclopropane, cyclobutane, cyclopentane, cyclohexane, cycloheptane.

In a preferred embodiment, the polymer of a carrier moiety POL of formula (Ia) and (Ib) comprises a polymer selected from the group of polymers consisting of polypeptides, 2-methacryloyl-oxyethyl phosphoyl cholins, hydrogels, PEG-based hydrogels, hyaluronic acid-based hydrogels, poly(acrylic acids), poly(acrylates), poly(acrylamides), poly(alkyloxy) polymers, poly(amides), poly(amidoamines), poly(amino acids), poly(anhydrides), poly(aspartamides), poly(butyric acids), poly(glycolic acids), polybutylene terephthalates, poly(caprolactones), poly(carbonates), poly(cyanoacrylates), poly(dimethylacrylamides), poly(esters), poly(ethylenes), poly(ethyleneglycols), poly(ethylene oxides), poly(ethyl phosphates), poly(ethyloxazolines), poly(glycolic acids), poly(hydroxyethyl acrylates), poly(hydroxyethyloxazolines), poly(hydroxymethacrylates), poly(hydroxypropylmethacrylamides), poly(hydroxypropyl methacrylates), poly(hydroxypropyloxazolines), poly(iminocarbonates), poly(lactic acids), poly(lactic-co-glycolic acids), poly(methacrylamides), poly(methacrylates), poly(methyloxazolines), poly(organophosphazenes), poly(ortho esters), poly(oxazolines), poly(propylene glycols), poly(siloxanes), poly(urethanes), poly(vinyl alcohols), poly(vinyl amines), poly(vinylmethylethers), poly(vinylpyrrolidones), silicones, celluloses, carbomethyl celluloses, hydroxypropyl methylcelluloses, chitins, chitosans, dextrans, dextrins, gelatins, hyaluronic acids and derivatives, functionalized hyaluronic acids, mannans, pectins, rhamnogalacturonans, starches, hydroxyalkyl starches, hydroxyethyl starches and other carbohydrate-based polymers, xylans, and copolymers thereof.

In one embodiment a carrier moiety POL of formula (Ia) and (Ib) of the carrier-linked prodrugs of the present invention is water-insoluble. In such case it is preferred that a moiety POL comprises, preferably consists of a crosslinked polymer, more preferably a crosslinked hydrogel. Preferred hydrogels are PEG-based hydrogels or hyaluronic acid-based hydrogels. Most preferably, a moiety POL of formula (Ia) and (Ib) comprises, preferably consists of a hydrogel as disclosed in WO-A 2006/003014 or WO-A2011/012715, which are enclosed herewith by reference.

In another embodiment a moiety POL of formula (Ia) and (Ib) comprises, preferably consists of a water-soluble polymer.

If a carrier moiety POL of formula (Ia) and (Ib) comprises, preferably consists of a water-soluble polymer, it is preferred that POL has a molecular weight of at least 0.2 kDa, such as from 0.2 kDa to 160 kDa, preferably POL has a molecular weight of from 2 kDa to 100 kDa, more preferably, POL has a molecular weight of from 5 kDa to 80 kDa and most preferably POL has a molecular weight of from 10 to 40 kDa.

In a preferred embodiment, a carrier moiety POL of formula (Ia) has the structure of formula (II):

Hyp¹ₘ - POL^{x} - Hyp² (II),

wherein
POL^{x} is a polymeric moiety having a molecular weight ranging from 0.5 kDa to 160 kDa,
Hyp¹ and Hyp² are independently a hyperbranched moiety, and
m is 0 or 1.

Functional groups of Hyp¹ and Hyp² are connected to moieties L of formula (Ia).

A polymeric moiety POL^{x} of formula (II) has a molecular weight of from 0.5 kDa to 160 kDa, preferably of from 2 kDa to 80 kDa and more preferably of from 5 kDa to 40kDa.

POL^{x} of formula (II) may be selected from the group of polymers consisting of, for example, polypeptides, 2-methacryloyl-oxyethyl phosphoyl cholins, water-soluble hydrogels, water-soluble PEG-based hydrogels, water-soluble hyaluronic acid-based hydrogels, poly(acrylic acids), poly(acrylates), poly(acrylamides), poly(alkyloxy) polymers, poly(amides), poly(amidoamines), poly(amino acids), poly(anhydrides), poly(aspartamides), poly(butyric acids), poly(glycolic acids), polybutylene terephthalates, poly(caprolactones), poly(carbonates), poly(cyanoacrylates), poly(dimethylacrylamides), poly(esters), poly(ethylenes), poly(ethyleneglycols), poly(ethylene oxides), poly(ethyl phosphates), poly(ethyloxazolines), poly(glycolic acids), poly(hydroxyethyl acrylates), poly(hydroxyethyloxazolines), poly(hydroxymethacrylates), poly(hydroxypropylmethacrylamides), poly(hydroxypropyl methacrylates), poly(hydroxypropyloxazolines), poly(iminocarbonates), poly(lactic acids), poly(lactic-co-glycolic acids), poly(methacrylamides), poly(methacrylates), poly(methyloxazolines), poly(organophosphazenes), poly(ortho esters), poly(oxazolines), poly(propylene glycols), poly(siloxanes), poly(urethanes), poly(vinyl alcohols), poly(vinyl amines), poly(vinylmethylethers), poly(vinylpyrrolidones), silicones, celluloses, carbomethyl celluloses, hydroxypropyl methylcelluloses, chitins, chitosans, dextrans, dextrins, gelatins, hyaluronic acids and derivatives, functionalized hyaluronic acids, mannans, pectins, rhamnogalacturonans, starches, hydroxyalkyl starches, hydroxyethyl starches and other carbohydrate-based polymers, xylans, and copolymers thereof.

The polymeric moiety POL^{x} of formula (II) may comprise a linear or branched polymer. Preferably, POL^{x} comprises, in particular consists of a linear polymer.

In one preferred embodiment, POL^{x} of formula (II) comprises, in particular consists of a PEG-based polymer or a poly(oxazoline)-based polymer; more preferably a linear PEG-based polymer. Even more preferably, POL^{x} of formula (II) consists of a PEG-based linear polymer.

If m in formula (II) is 0, it is preferred that POL^{x} comprises, preferably consists of a structure of the formula

X1-(OCH₂CH₂)ₚ-O-(CH₂)ₙ-X2-,

wherein
n is 1, 2, 3, or 4, preferably n is 1, 2, or 3, and more preferably 2 or 3;
p is an integer from 5 to 2000, preferably p is an integer from 10 to 1000, more preferably p is an integer from 100 to 1000;
X2 is a functional group covalently linked to Hyp²; and
X1 is selected from H, CH₃ and C₂H₅.

If m in formula (II) is 1, it is preferred that POL^{x} comprises, preferably consists of a structure of the formula

-X3-(CH₂)ₙ₁-(OCH₂CH₂)ₚ-O-(CH₂)ₙ₂-X2-,

wherein
n1 and n2 are independently 1, 2, 3, or 4, preferably n1 and n2 are independently 1, 2, or 3, more preferably 2 or 3;
p is an integer from 5 to 2000, preferably p is an integer from 10 to 1000, more preferably p is an integer from 100 to 1000; and
X2 and X3 are independently a functional group covalently linked to Hyp¹ and Hyp², respectively.

In a preferred embodiment m in formula (II) is 0.

In another preferred embodiment, POL^{x} of formula (II) is a polypeptide (or protein), in particular a non-immunogenic polypeptide as described below.

Preferably, a polymeric moiety POL^{x} of formula (II) is a polypeptide which comprises at least about 100 amino acid residues, in particular which consists of at least about 100 amino acid residues. In a preferred embodiment, amino acids selected from alanine, serine and/or proline residues are present, in particular are mainly present, and which polypeptide moiety preferably has a random coil conformation at physiological conditions. It is understood that such a polypeptide moiety POL^{x} of formula (II) may transiently or temporarily not form a random coil, for example when present in a lyophilisate or dried composition.

A polypeptide moiety POL^{x} of formula (II) may have a random coil conformation with an amino acid sequence consisting of maximally about 3000 amino acid residues, preferably of maximally about 900 amino acid residues, more preferably of maximally about 800 amino acid residues, even more preferably of maximally about 700 amino acid residues, particularly preferably of maximally about 600 amino acid residues. Thus, the amino acid sequence forming random coil conformation may consist of maximally about 500 amino acid residues or of maximally about 450 amino acid residues.

In particular embodiments said amino acid sequence forming random coil conformation consists of about 100 to 1000 amino acid residues as characterized herein, i.e. comprising alanine, serine and/or proline as main or unique residues as defined below.

In a preferred embodiment, a polypeptide moiety POL^{x} of formula (II) consists mainly of one, two or three, preferably three of the amino acid residues alanine, serine and proline, whereby proline residues represent preferably about 4 % to about 40 % of the polypeptide moiety POL^{x} of formula (II). The alanine and serine residues comprise the remaining at least 60 % to 96 % of the polypeptide moiety POL^{x} of formula (II). However, as will be detailed herein below said polypeptide moiety POL^{x} of formula (II) may also comprise further amino acids differing from alanine, serine, and proline, i.e. as minor constituents.

The term "minor constituent" as used herein means that maximally 10 % (i.e. maximally 10 of 100 amino acids) may be different from alanine, serine and proline, preferably maximally 8 % (i.e. maximally 8 of 100 amino acids) may be different than alanine, serine and proline, more preferably maximally 6 % (i.e. maximally 6 of 100 amino acids) may be different from alanine, serine and proline, even more preferably maximally 5 % (i.e. maximally 5 of 100 amino acids) may be different from alanine, serine and proline, particularly preferably maximally 4 % (i.e. maximally 4 of 100 amino acids) may be different from alanine, serine and proline, more particularly preferably maximally 3 % (i.e. maximally 3 of 100 amino acids) may be different from alanine, serine and proline, even more particularly preferably maximally 2 % (i.e. maximally 2 of 100 amino acids) may be different from alanine, serine and proline and most preferably maximally 1 % (i.e. maximally 1 of 100 of the amino acids) may be different from alanine, serine and proline. Said amino acids different from alanine, serine and proline may be selected from the group consisting of different from alanine, serine and proline may be selected from the group of natural or proteinogenic amino-acids comprising Arg, Asn, Asp, Cys, Gln, Glu, Gly, His, Ile, Leu, Lys, Met, Phe, Thr, Trp, Tyr, Val, selenocystein, selenomethionin, and hydroxyproline. Minor constituents may also be selected from non-naturally occurring amino acids.

The term "at least about 100/150/200/250/300/300/350 (etc) amino acid residues" is not limited to the concise number of amino acid residues but also comprises amino acid stretches that comprise an additional 10 % to 20 % or comprise 10 % to 20 % less residues. For example "at least about 100 amino acid residues" may also encompass 80 to 100 and about 100 to 120 amino acid residues without deferring from the gist of the present invention.

In one embodiment, the polypeptide moiety POL^{x} of formula (II) comprises a plurality of polymer cassettes wherein said polymer cassettes consist of one, two or three of the amino acids selected from Ala, Ser, and Pro and wherein no more than 6 consecutive amino acid residues are identical and wherein said proline residues constitute more than 4 % and less than 40 % of the amino acids of said polypeptide moiety POL^{x} of formula (II).

A polypeptide moiety POL^{x} of formula (II) may comprise a plurality, in particular 2, 3, 4, 5 or more of identical polymer cassettes or a plurality of non-identical polymer cassettes. Nonlimiting examples of polymer cassettes consisting of Ala, Ser and Pro residues are provided herein below; see SEQ ID NO:9, SEQ ID NO:10, SEQ ID NO:11, SEQ ID NO:12, SEQ ID NO:13 and SEQ ID NO:14 or peptide fragments or multimers of these sequences. A polymer cassette may consist of at least 3, 4, 5, 6, 7, 8, 9, 10, 11, 12, 13, 14, 15, 16, 17, 18, 19, 20, 21, 22, 23, 24, 25, 26, 27, 28, 29, 30 or more amino acid residues, wherein each polymer cassette comprises (an) Ala, Ser, and Pro residue(s).

In one embodiment, such polymer cassette does not comprise more than 100 amino acid residues. Preferably, a polymer cassette as defined herein comprises more than about 4 %, preferably more than about 5 %, even more preferably more than about 6%, particularly preferably more than about 8 %, more particularly preferably more than about 10 %, even more particularly preferably more than about 15 % and most preferably more than about 20 % proline residues. Such polymer cassette as defined herein preferably comprises less than about 40 % or less than about 35 % proline residues.

In one preferred embodiment the polypeptide moiety POL^{x} of formula (II) comprises, in particular consists of formula (a):

Serₓ[Ala_{y} Ser_{z}]ₙ (a),

which formula further comprises proline residues as defined herein and wherein
x is independently selected from integer 0 to 6,
each y is independently selected from integer ranging of from 1 to 6,
each z is independently selected from integer ranging of from 1 to 6.
n is any integer so that a polypeptide moiety POL^{x} of formula (II) consists of at least about 100 amino acid residues, and in particular of at least about 100 to about 3000 amino acid residues, preferably to about 2000 and more preferably to about 1000 amino acid residues.

In one embodiment, all y of formula (a) and z of formula (a) of the n Ala_{y} Ser_{z} monomer moieties of formula (a) are identical. In another embodiment, the y of formula (a) and z of formula (a) of the n Ala_{y} Ser_{z} monomer moieties of formula (a) are different.

In another preferred embodiment, a polypeptide moiety POL^{x} of formula (II) comprises no more than 5 identical consecutive amino acid residues, more preferably no more than 4 identical consecutive amino acid residues and most preferably no more than 3 identical consecutive amino acid residues.

As already indicated herein above, a polypeptide moiety POL^{x} of formula (II) comprises in one embodiment proline residues, wherein said proline residues constitute more than about 4 %, preferably more than about 5 %, even more preferably more than about 6 %, particularly preferably more than about 8 %, more particularly preferably more than about 10 %, even more particularly preferably more than about 15 % and most preferably more than about 20 % of the amino acids of POL^{x} of formula (II).

In another preferred embodiment, a polypeptide moiety POL^{x} of formula (II) comprises more than about 4 % but less than about 50 %, preferably more than about 10 % but less than about 50 % and most preferably more than about 20 % but less than about 50 % alanine residues of the amino acids constituting the polypeptide moiety POL^{x} of formula (II).

In a further preferred embodiment, a polypeptide moiety POL^{x} of formula (II) comprises more than about 4 % and less than about 50 %, preferably more than about 10 % but less than about 50 % and most preferably more than about 20 % but less than about 50 % serine residues of the amino acids constituting the polypeptide moiety POL^{x} of formula (II).

Preferably, a polypeptide moiety POL^{x} of formula (II) comprises about 35 % proline residues, about 50 % alanine residues and about 15 % serine residues of the amino acids constituting the polypeptide moiety POL^{x} of formula (II). Alternatively, a polypeptide moiety POL^{x} of formula (II) may comprise about 35 % proline residues, about 15 % alanine residues and about 50 % serine residues of the amino acids constituting the polypeptide moiety POL^{x} of formula (II).

Preferably, a polypeptide moiety POL^{x} of formula (II) comprises one or more of the following alanine-serine polymer cassettes:
SEQ ID NO:1
   AAAASSASSASSSSSAAASA
SEQ ID NO:2
   AASAAASSAAASAAAASASS
SEQ ID NO:3
   ASASASASASASSAASAASA
SEQ ID NO:4
   SAASSSASSSSAASSASAAA
SEQ ID NO:5
   SSSSAASAASAAAAASSSAS
SEQ ID NO:6
   SSASSSAASSSASSSSASAA
SEQ ID NO:7
   SASASASASASAASSASSAS
   and
SEQ ID NO:8
   ASSAAASAAAASSAASASSS.

The multimers of these alanine-serine polymer cassettes may form random coil conformation in case the resulting amino acid sequence further comprises proline residues as defined herein above.

In a preferred embodiment, the polypeptide moiety POL^{x} of formula (II) comprises one or more of the following polymer cassettes:
SEQ ID NO:9
   ASPAAPAPASPAAPAPSAPA
SEQ ID NO: 10
   AAPASPAPAAPSAPAPAAPS
SEQ ID No:11
   APSSPSPSAPSSPSPASPSS
   and
SEQ ID NO:15
   SAPSSPSPSAPSSPSPASPS.

SEQ ID NO:15 corresponds to the herein provided SEQ ID No:11 in a circularly permuted form, wherein the last serine was removed and another serine was appended as starting amino acid. As a consequence, multimers of this modified sequence possess essentially the same internal repeating unit as multimers of the non-modified sequence, except for the very first and the very last residue. Accordingly, SEQ ID NO:15 may be considered as an example of a further polymer cassette for a polypeptide moiety POL^{x} of formula (II). It is clear for the person skilled in the art that also other polymer cassettes and (shorter) peptide fragments or circularly permuted versions of the herein provided amino acid polymers may be used as polymer cassettes for a polypeptide moiety POL^{x} of formula (II).

Yet, even further and illustrative amino acid polymers forming random coil conformation may comprise amino acid sequences that may be selected from the group consisting of the following sequences:
SEQ ID NO:12
   SSPSAPSPSSPASPSPSSPA
SEQ ID NO:13
   AASPAAPSAPPAAASPAAPSAPPA
   and
SEQ ID NO:14
   ASAAAPAAASAAASAPSAAA.

Therefore, preferred polymer cassettes for a polypeptide moiety POL^{x} of formula (II) are selected from the following sequences:
ASPAAPAPASPAAPAPSAPA (SEQ ID NO:9),
AAPASPAPAAPSAPAPAAPS (SEQ ID NO:10),
APSSPSPSAPSSPSPASPSS (SEQ ID NO:11),
SSPSAPSPSSPASPSPSSPA (SEQ ID NO:12),
AASPAAPSAPPAAASPAAPSAPPA (SEQ ID NO:13), and
ASAAAPAAASAAASAPSAAA (SEQ ID NO:14);
or circular permuted versions or (a) multimer(s) of these sequences as a whole or parts of these sequences.

Again, also (a) peptide fragment(s) or (a) multimer(s) or circularly permuted versions of these sequences and the sequences provided herein above may be employed in the context of the present invention as polymer cassettes for a polypeptide moiety POL^{x} of formula (II). The person skilled in the art is readily in a position to generate further amino acid polymer cassettes that form random coil conformation under physiological conditions and are constituted of mainly alanine, serine, and proline as defined herein. Such other and further examples of random coil conformation forming amino acid polymer cassettes to be used for a polypeptide moiety POL^{x} of formula (II) may, inter alia, comprise combinations and/or peptide fragments or circularly permuted versions of the specific polymer cassettes shown above.

Accordingly, the exemplified polymer cassettes may also provide for individual peptide fragments which may be newly combined to form further polymer cassettes.

In accordance with the above, a polypeptide moiety POL^{x} of formula (II) may comprise a multimer of sequences consisting of either one of the amino acid sequences with SEQ ID NO:9, 10, 11, 12, 13 or 14 as disclosed herein above or may comprise a multimer of sequences consisting of more than one of amino acid sequences SEQ ID NO:9, 10, 11, 12, 13 and 14. Furthermore, it is envisaged that also peptide fragments or circularly permuted versions of these exemplified sequences may be used to build up further polymer cassettes of a polypeptide moiety POL^{x} of formula (II).

In another embodiment, a polypeptide moiety POL^{x} of formula (II) may comprise a multimer of sequences consisting of a (circular) permutation of the amino acid sequence selected from the group consisting of SEQ ID NOs:9, 10, 11, 12, 13, 14, 15 and (a) multimers(s) of these (circular) permutated sequences.

In yet another embodiment, a polypeptide moiety POL^{x} of formula (II) may comprise a multimer consisting of a peptide fragment/part of the amino acid sequence selected from the group consisting of SEQ ID NO: 9, 10, 12, 13, 14, 15 and (a) multimers(s) of these exemplified polymer cassettes.

Peptide fragments of these sequences to be employed for the generation of a polypeptide moiety POL^{x} of formula (II) may consist of at least 3, preferably of at least 4, more preferably of at least 5, even more preferably of at least 6, still more preferably of at least 8, particularly preferably of at least 10, more particularly preferably of at least 12, even more particularly preferably of at least 14, preferably of at least 6, still more preferably of at least 8, particularly preferably of at least 10, more particularly preferably of at least 12, even more particularly preferably of at least 14, even more particularly preferably of at least 16, and most preferably of at least 18 consecutive amino acids of the amino acid sequence selected from the group consisting of said SEQ ID NOs: 9, 10, 11, 12, 13 and 14.

For example, individual peptide fragments of the inventive polymer cassettes may be combined to further individual polymer cassettes as long as the above-identified rules for the overall distribution and amount of alanine, serine and proline are respected. Again, these polymer cassettes may also comprise further amino acid residues, however only as minimal or minor constituents, i. e. maximally 10 %, preferably maximally 2 % of the individual polymer cassette. POL^{x} moieties of formula (II) comprising polymer cassettes consist, in one embodiment of the present invention, of at least about 100 amino acid residues. Individual polymer cassettes may be combined in order to form longer random coil forming amino acid polymers, whereby a maximal length of a polypeptide moiety POL^{x} of formula (II) is about 3000 amino acids.

Preferably, POL^{x} of formula (II) is covalently linked to Hyp¹ and Hyp², in particular by a permanent linkage, more preferably by a permanent amide linkage.

In preferred carrier-linked prodrugs of formula (Ia) and (Ib) functional groups of Hyp¹ and Hyp² of formula (II) are connected to at least one reversible prodrug linker moiety of formula (Ic).

In a preferred embodiment, each moiety Hyp¹ and each moiety Hyp² of formula (II) independently comprises, preferably consists of, a moiety selected from
- a polyalcohol in bound form comprising at least 2 hydroxyl groups (preferably further comprising a functional group, which is preferably an additional amine group or a carboxylic acid group, more preferably an additional carboxylic acid group),
   preferably selected from glycerol, pentaerythritol, dipentaerythritol, tripentaerythritol, hexaglycerine, sucrose, sorbitol, fructose, mannitol, glucose, cellulose, amyloses, starches, hydroxyalkyl starches, polyvinylalcohols, dextranes, and hyualuronans,
- or a polyamine in bound form comprising at least 2 amine groups (preferably further comprising a functional group, which is preferably an additional hydroxyl group or a carboxylic acid group, more preferably a carboxylic acid group),
   preferably selected from ornithine, diornithine, triornithine, tetraornithine, pentaornithine, hexaornithine, heptaornithine, octaornithine, nonaornithine, decaornithine, undecaornithine, dodecaornithine, tridecaornithine, tetradecaornithine, pentadecaornithine, hexadecaornithine, heptadecaornithine, octadecaornithine, nonadecaornithine, diaminobutyric acid, di(diaminobutyric acid), tri(diaminobutyric acid), tetra(diaminobutyric acid), penta(diaminobutyric acid), hexa(diaminobutyric acid), hepta(diaminobutyric acid), octa(diaminobutyric acid), nona(diaminobutyric acid), deca(diaminobutyric acid), undeca(diaminobutyric acid), dodeca(diaminobutyric acid), trideca(diaminobutyric acid), tetradeca(diaminobutyric acid), pentadeca(diaminobutyric acid), hexadeca(diaminobutyric acid), heptadeca(diaminobutyric acid), octadeca(diaminobutyric acid), nonadeca(diaminobutyric acid), lysine, dilysine, trilysine, tetralysine, pentalysine, hexalysine, heptalysine, octalysine, nonalysine, decalysine, undecalysine, dodecalysine, tridecalysine, tetradecalysine, pentadecalysine, hexadecalysine, heptadecalysine, octadecalysine, nonadecalysine, oligolysines, triornithine, tetraornithine, pentaornithine, hexaornithine, heptaornithine, octaornithine, nonaornithine, decaornithine, undecaornithine, dodecaornithine, tridecaornithine, tetradecaornithine, pentadecaornithine, hexadecaornithine, heptadecaornithine, octadecaornithine, nonadecaornithine, tridiaminobutyric acid, tetradiaminobutyric acid, pentadiaminobutyric acid, hexadiaminobutyric acid, heptadiaminobutyric acid, octadiaminobutyric acid, nonadiaminobutyric acid, decadiaminobutyric acid, undecadiaminobutyric acid, dodecadiaminobutyric acid, tridecadiaminobutyric acid, tetradecadiaminobutyric acid, pentadecadiaminobutyric acid, hexadecadiaminobutyric acid, heptadecadiaminobutyric acid, octadecadiaminobutyric acid, nonadecadiaminobutyric acid,
- or a polycarboxylate in bound form comprising at least 2 carboxylate groups, (preferably further comprising a functional group, which is preferably an additional amine group or a hydroxyl group, more preferably an additional amine group),
   preferably selected from di(glutamic acid), tri(glutamic acid), tetra(glutamic acid), penta(glutamic acid), hexa(glutamic acid), hepta(glutamic acid), octa(glutamic acid), nona(glutamic acid), deca(glutamic acid), undeca(glutamic acid), dodeca(glutamic acid), trideca(glutamic acid), tetradeca(glutamic acid), pentadeca(glutamic acid), hexadeca(glutamic acid), heptadeca(glutamic acid), octadeca(glutamic acid), nonadeca(glutamic acid), di(aspartic acid), tri(aspartic acid), tetra(aspartic acid), penta(aspartic acid), hexa(aspartic acid), hepta(aspartic acid), octa(aspartic acid), nona(aspartic acid), deca(aspartic acid), undeca(aspartic acid), dodeca(aspartic acid), trideca(aspartic acid), tetradeca(aspartic acid), pentadeca(aspartic acid), hexadeca(aspartic acid), heptadeca(aspartic acid), octadeca(aspartic acid), nonadeca(aspartic acid), polyethyleneimines, and polyvinylamines.

More preferably, each moiety Hyp¹ and each moiety Hyp² of formula (II) are independently selected from the group comprising, in particular consisting of, in bound form, dilysine, trilysine, tetralysine, pentalysine, hexalysine, heptalysine, octalysine, nonalysine, decalysine, undecalysine, dodecalysine, tridecalysine, tetradecalysine, pentadecalysine, hexadecalysine, heptadecalysine, octadecalysine, nonadecalysine, triornithine, tetraornithine, pentaornithine, hexaornithine, heptaornithine, octaornithine, nonaornithine, decaornithine, undecaornithine, dodecaornithine, tridecaornithine, tetradecaornithine, pentadecaornithine, hexadecaornithine, heptadecaornithine, octadecaornithine, nonadecaornithine, tridiaminobutyric acid, tetradiaminobutyric acid, pentadiaminobutyric acid, hexadiaminobutyric acid, heptadiaminobutyric acid, octadiaminobutyric acid, nonadiaminobutyric acid, decadiaminobutyric acid, undecadiaminobutyric acid, dodecadiaminobutyric acid, tridecadiaminobutyric acid, tetradecadiaminobutyric acid, pentadecadiaminobutyric acid, hexadecadiaminobutyric acid, heptadecadiaminobutyric acid, octadecadiaminobutyric acid, nonadecadiaminobutyric acid, di(glutamic acid), tri(glutamic acid), tetra(glutamic acid), penta(glutamic acid), hexa(glutamic acid), hepta(glutamic acid), octa(glutamic acid), nona(glutamic acid), deca(glutamic acid), undeca(glutamic acid), dodeca(glutamic acid), trideca(glutamic acid), tetradeca(glutamic acid), pentadeca(glutamic acid), hexadeca(glutamic acid), heptadeca(glutamic acid), octadeca(glutamic acid), nonadeca(glutamic acid), di(aspartic acid), tri(aspartic acid), tetra(aspartic acid), penta(aspartic acid), hexa(aspartic acid), hepta(aspartic acid), octa(aspartic acid), nona(aspartic acid), deca(aspartic acid), undeca(aspartic acid), dodeca(aspartic acid), trideca(aspartic acid), tetradeca(aspartic acid), pentadeca(aspartic acid), hexadeca(aspartic acid), heptadeca(aspartic acid), octadeca(aspartic acid), nonadeca(aspartic acid), polyethyleneimines, and low-molecular weight PEI.

Even more preferably, each moiety Hyp¹ and each moiety Hyp² of formula (II) are independently selected from the group comprising, more preferably consisting of, in bound form, trilysine, tetralysine, pentalysine, hexalysine, heptalysine, octalysine, nonalysine, decalysine, undecalysine, dodecalysine, tridecalysine, tetradecalysine, pentadecalysine, hexadecalysine, and heptadecalysine, even more preferably a moiety Hyp of formula (I) comprises, preferably consists of, in bound form, trilysine, heptalysine or pentadecalysine.

More preferably, a moiety Hyp¹ and a moiety Hyp² of formula (II) are independently selected from any one of the following structures: 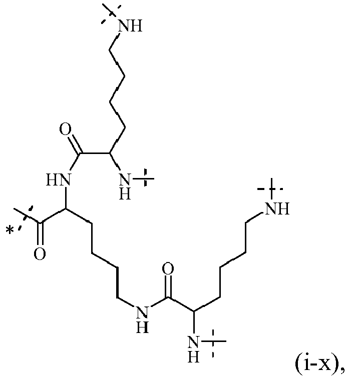 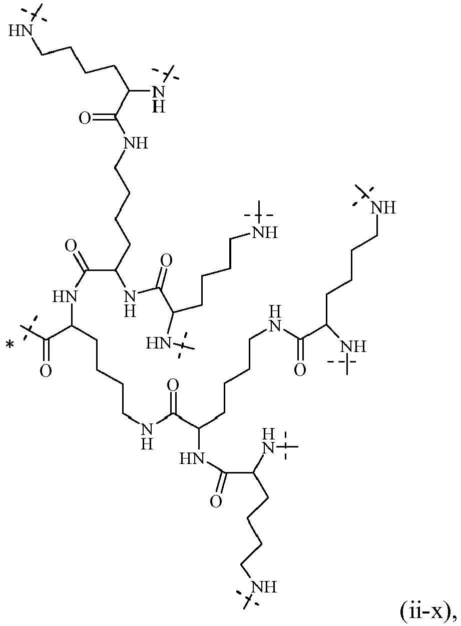 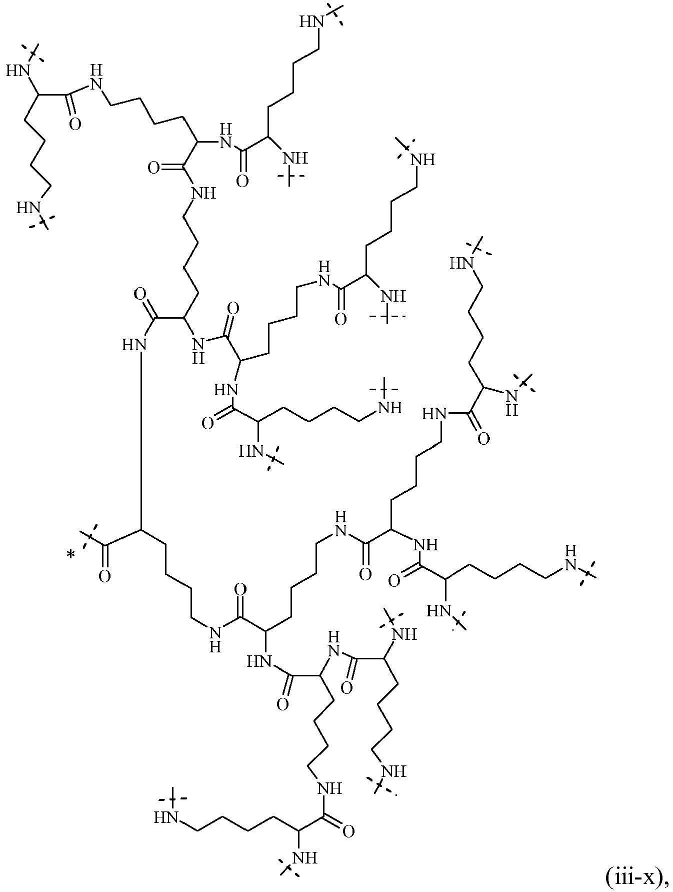 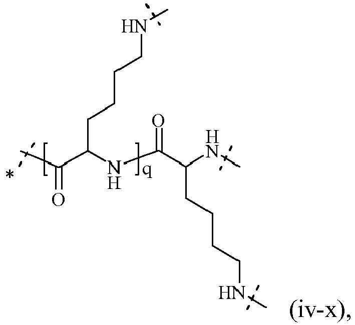 wherein
dashed lines marked with an asterisk indicate attachment to POL^{x} of formula (II) and unmarked dashed lines indicate attachment to a moiety L of formula (Ia); and
q is an integer from 0 to 15, in particular from 3 to 7.
More preferably, q is 6.

Preferably, Hyp¹ and Hyp² of formula (II) are each a heptalysinyl group, more preferably Hyp¹ and Hyp² of formula (II) each have the structure of formula (ii-x) above.

Preferably, Hyp¹ and Hyp² of formula (II) have the same structure.

Functional groups of Hyp¹ and Hyp² of formula (II) are connected to moieties L of formula (Ia) or (Ib). Remaining functional groups of Hyp¹ and Hyp² of formula (II) which are not connected to a moiety L may, independently of each other, be capped with suitable capping reagents or may optionally be connected to at least one targeting moiety, in particular through permanent linkages.

Therefore, in preferred embodiments of the carrier-linked prodrugs of the present invention moieties Hyp¹ and Hyp² of formula (II) are connected to POL^{x} of formula (II) and functional groups of Hyp¹ and Hyp² of formula (II) are connected to moieties L of formula (Ia), targeting moieties and/or capping groups.

In a preferred embodiment, all functional groups of a moiety Hyp¹ and of a moiety Hyp² of formula (II) are connected to moieties L.

Preferably, a moiety Hyp¹ and a moiety Hyp² of formula (II) have independently a molecular weight from 0.1 kDa to 4 kDa, more preferably from 0.4 kDa to 2 kDa. Preferably, a moiety Hyp¹ and a moiety Hyp² of formula (II) has each independently at least 3 branchings and are each independently conjugated to at least 4 moieties L, targeting moieties, and/or capping groups and each independently have at most 63 branchings and are each independently at most conjugated to 64 moieties L, targeting moieties, and/or capping groups. It is preferred that a moiety Hyp¹ and a moiety Hyp² of formula (II) has each independently at least 7 branchings and are each independently conjugated to at least 8 moieties L, targeting moieties, and/or capping groups and have each independently at most 31 branchings and are each independently at most conjugated to 32 moieties L, targeting moieties, and/or capping groups.

Preferably, a moiety Hyp¹ and a moiety Hyp² of formula (II) is each independently a hyperbranched peptide or polypeptide. Preferably, such hyperbranched peptide or polypeptide comprises lysine in bound form. Preferably, a moiety Hyp¹ and a moiety Hyp² of formula (II) independently has a molecular weight from 0.1 kDa to 4 kDa, in particular from 0.4 kDa to 2 kDa.

Preferably, m=0 and the sub-structure POL^{x}-Hyp² of formula (II) is selected from the following structures: 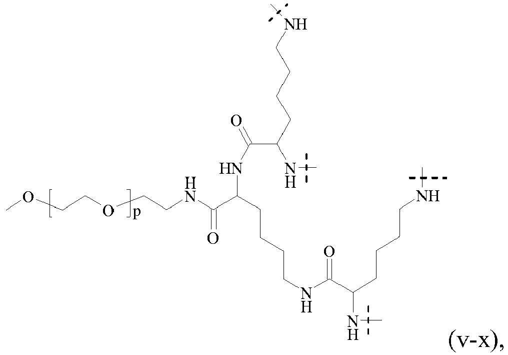 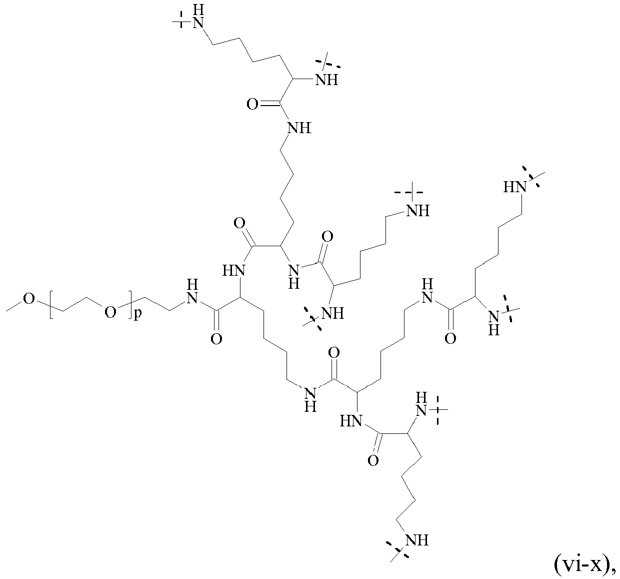 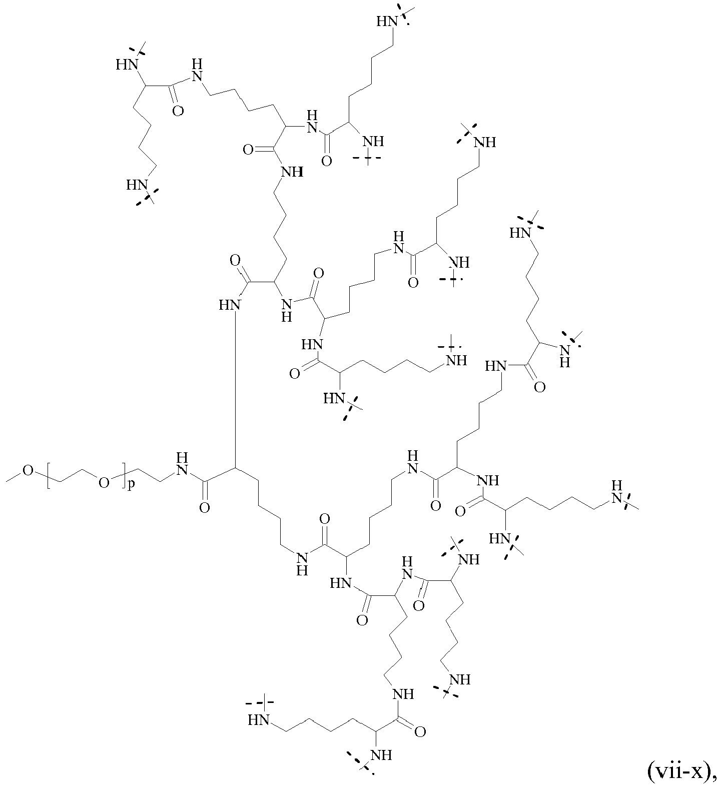 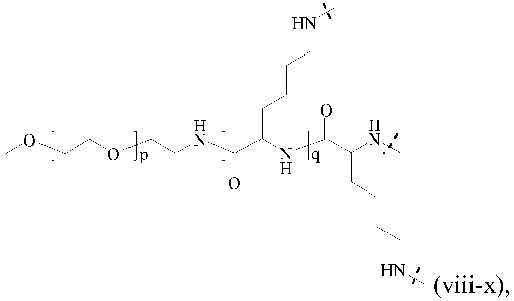 wherein
p is an integer of from 5 to 2000, preferably 10 to 1000, in particular 100 to 1000, and
q is an integer from 0 to 15, preferably from 3 to 7; more preferably, q is 6.

In another preferred embodiment the carrier moiety POL of formula (Ia) has the structure of formula (III):

B(̵A-Hyp^{y})ₙ (III),

wherein
B is a branching core,
each A is independently a poly(ethylene glycol)-based polymeric chain,
each Hyp^{y} is independently a branched moiety, and
n is an integer of from 3 to 32;

In a preferred embodiment, the branching core B of formula (III) comprises, preferably consists of, a moiety selected from:
- a polyalcohol comprising at least 2 hydroxyl groups (preferably further comprising a functional group, which is preferably an additional amino group or a carboxylic acid group, more preferably an additional carboxylic acid group),
   preferably B is selected from glycerol, pentaerythritol, dipentaerythritol, tripentaerythritol, hexaglycerine, sucrose, sorbitol, fructose, mannitol, glucose, cellulose, amyloses, starches, hydroxyalkyl starches, polyvinylalcohols, dextranes, and hyualuronans,
- or a polyamine comprising at least 2 amine groups (preferably further comprising a functional group, which is preferably an additional hydroxyl group or a carboxylic acid group, more preferably a carboxylic acid group),
   preferably selected from ornithine, diornithine, triornithine, tetraornithine, pentaornithine, hexaornithine, heptaornithine, octaornithine, nonaornithine, decaornithine, undecaornithine, dodecaornithine, tridecaornithine, tetradecaornithine, pentadecaornithine, hexadecaornithine, heptadecaornithine, octadecaornithine, nonadecaornithine, diaminobutyric acid, di(diaminobutyric acid), tri(diaminobutyric acid), tetra(diaminobutyric acid), penta(diaminobutyric acid), hexa(diaminobutyric acid), hepta(diaminobutyric acid), octa(diaminobutyric acid), nona(diaminobutyric acid), deca(diaminobutyric acid), undeca(diaminobutyric acid), dodeca(diaminobutyric acid), trideca(diaminobutyric acid), tetradeca(diaminobutyric acid), pentadeca(diaminobutyric acid), hexadeca(diaminobutyric acid), heptadeca(diaminobutyric acid), octadeca(diaminobutyric acid), nonadeca(diaminobutyric acid), lysine, dilysine, trilysine, tetralysine, pentalysine, hexalysine, heptalysine, octalysine, nonalysine, decalysine, undecalysine, dodecalysine, tridecalysine, tetradecalysine, pentadecalysine, hexadecalysine, heptadecalysine, octadecalysine, nonadecalysine, oligolysines, polyethyleneimines, and polyvinylamines;
wherein the polyalcohol or polyamine is in bound form.

In a preferred embodiment, the branching core B of formula (III) comprises, preferably consists of pentaerythritol.

Preferably, a poly(ethylene glycol)-based polymeric chain (PEG-based polymeric chain) A connected to the branching core B of formula (III) consists of a linear PEG chain, of which one terminus is connected to B of formula (III) and the other terminus is connected to Hyp^{y} of formula (III). It is understood that a PEG-based chain A of formula (III) may optionally be terminated in case of a branched PEG chain and/or may optionally be interrupted in case of a branched or linear PEG chain by alkyl or aryl groups and may optionally be substituted with heteroatoms and/or functional groups.

Each of the n sub-structures A-Hyp^{y} of formula (III) extending from the branching core B of formula (III) may be independently of each other the same or different sub-structures A-Hyp^{y}. In a preferred embodiment, the n sub-structures A-Hyp^{y} of formula (III) are the same moieties.

Each A and each Hyp^{y} of formula (III) may be selected independently from the other moieties A and Hyp^{y} of the carrier moiety of formula (III). Preferably, all n sub-structures A-Hyp^{y} connected to B of formula (III) have an identical structure.

Preferably, the PEG-based polymeric chains A of formula (III) are connected to B through permanent linkages.

n of formula (III) is an integer from 3 to 32. Preferably, n is an integer from 3 to 16, more preferably n is an integer from 4 to 8 and most preferably n is 4.

In a preferred embodiment n of formula (III) is 4 and m of formula (II) is 2.

In one embodiment, a PEG-based polymeric chains A of formula (III) is selected from linear and branched PEG-based polymeric chains. Preferably, each A is a linear PEG-based polymeric chain.

Preferably, each A of formula (III) is independently selected from the formula

-X3-(CH₂)ₙ₁-(OCH₂CH₂)ₚ-O-(CH₂)ₙ₂-X2,

wherein
n1 and n2 are independently 1, 2, 3, or 4, preferably n1 and n2 are independently 1, 2, or 3, more preferably 2 or 3;
p is an integer from 5 to 2000, preferably p is an integer from 10 to 1000, more preferably p is an integer from 100 to 1000; and
X3 is a a chemical bond or linkage group covalently linked to B, and
X2 is a chemical bond or linkage group covalently linked to a moiety Hyp^{y}.

Preferably, a linkage between a moiety A and a moiety Hyp^{y} of formula (III) is a permanent linkage, more preferably a permanent linkage comprising a linkage group comprising, in particular consisting of a group selected from amine groups, amide groups, carbamate groups, thioether groups, ether groups, and most preferably the permanent linkage between a moiety A and a moiety Hyp^{y} of formula (III) is an amide linkage.

In a preferred embodiment, the sub-structure B(̵A)ₙ of formula (III) is a multi-arm PEG derivative as, for instance, detailed in the products list of JenKem Technology, USA (accessed by download from http://jenkemusa.net/pegproducts2.aspx on March 8, 2011), such as a 4-arm-PEG derivative, in particular comprising a pentaerythritol core, an 8-arm-PEG derivative comprising a hexaglycerin core, and an 8-arm-PEG derivative comprising a tripentaerythritol core. Most preferred are sub-structures B(̵A)ₙ of formula (III) comprising, in particular consisting of, moieties selected from:
a 4-arm PEG Amine comprising a pentaerythritol core: 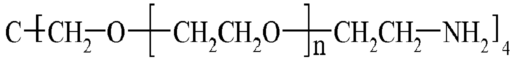 with n ranging from 400 to 2000;
a 4-arm PEG Carboxyl comprising a pentaerythritol core: 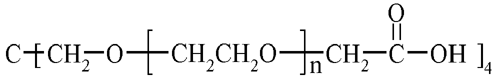 with n ranging from 400 to 2000;
an 8-arm PEG Amine comprising a hexaglycerin core: 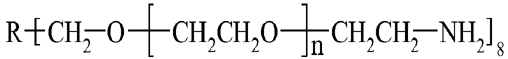
   with n ranging from 400 to 2000 and
   R = hexaglycerin core structure;
an 8-arm PEG Carboxyl comprising a hexaglycerin core: 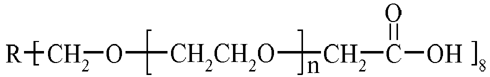
   with n ranging from 400 to 2000 and
   R = hexaglycerin core structure;
an 8-arm PEG Amine comprising a tripentaerythritol core: 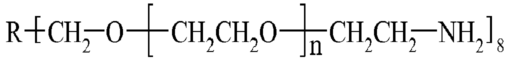
   with n ranging from 400 to 2000
   and R = tripentaerythritol core structure;
   and an 8-arm PEG Carboxyl comprising a tripentaerythritol core: 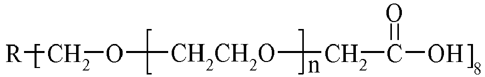
   with n ranging from 400 to 2000 and
   R = tripentaerythritol core structure;
each in bound form.

In a preferred embodiment, the molecular weight of a sub-structure B(̵A)ₙ of formula (III) ranges from 1 kDa to 80 kDa, more preferably from 1 kDa to 40 kDa and even more preferably from10 kDa to 40 kDa. It is understood that the terminal amine groups or carboxyl groups, respectively, are used for conjugation to a moiety Hyp^{y} of formula (III).

Functional groups of a branched moiety Hyp^{y} of formula (III) are connected to moieties L of formula (Ia).

In a preferred embodiment, a moiety Hyp^{y} of formula (III) is connected to a moiety L of formula (Ia) or (IA) through a functional group selected from amide groups, carbamate groups, ester groups, ether groups, amine groups, thioether groups. Preferably, Hyp^{y} of formula (III) is connected to a moiety L of formula (Ia) through amide groups, thioether groups and/or ether groups, even more preferably through amide groups.

Optionally, functional groups of Hyp^{y} of formula (III) which are not connected to a moiety L of formula (Ia) may be capped with suitable capping reagents and/or may optionally be connected to at least one targeting moiety, in particular through permanent linkages. Therefore, a moiety Hyp^{y} of formula (III) may be connected to moieties L of formula (Ia), capping moieties and/or targeting moieties. Preferably, Hyp^{y} of formula (III) is connected to moieties L of formula (Ia) and is not connected to capping moieties and/or targeting moieties. Targeting moieties, if present, may be conjugated to Hyp^{y} of formula (III) either directly or indirectly through spacer moieties.

Examples of suitable capping moieties are linear, branched or cyclic C₁₋₈ alkyl groups.

In one embodiment, each branched moiety Hyp^{y} of formula (III) is connected to at least two moieties L of formula (Ia). More preferably, each branched moiety Hyp^{y} of formula (III) is connected to at least three moieties L of formula (Ia). Most preferably, each branched moiety Hyp^{y} of formula (III) is connected to at least four moieties L of formula (Ia).

A branched moiety Hyp^{y} of the carrier of formula (III) comprises, preferably consists of preferably consists of, a moiety selected from
- a polyalcohol in bound form comprising at least 2 hydroxyl groups (preferably further comprising a functional group, which is preferably an additional hydroxyl group or a carboxylic acid group, more preferably an additional hydroxyl group),
   preferably selected from glycerol, pentaerythritol, dipentaerythritol, tripentaerythritol, hexaglycerine, sucrose, sorbitol, fructose, mannitol, glucose, cellulose, amyloses, starches, hydroxyalkyl starches, polyvinylalcohols, dextranes, and hyualuronans,
- or a polyamine in bound form comprising at least 2 amine groups (preferably further comprising a functional group, which is preferably an additional amine group or a carboxylic acid group, more preferably a carboxylic acid group),
   preferably selected from ornithine, diornithine, triornithine, tetraornithine, pentaornithine, hexaornithine, heptaornithine, octaornithine, nonaornithine, decaornithine, undecaornithine, dodecaornithine, tridecaornithine, tetradecaornithine, pentadecaornithine, hexadecaornithine, heptadecaornithine, octadecaornithine, nonadecaornithine, diaminobutyric acid, di(diaminobutyric acid), tri(diaminobutyric acid), tetra(diaminobutyric acid), penta(diaminobutyric acid), hexa(diaminobutyric acid), hepta(diaminobutyric acid), octa(diaminobutyric acid), nona(diaminobutyric acid), deca(diaminobutyric acid), undeca(diaminobutyric acid), dodeca(diaminobutyric acid), trideca(diaminobutyric acid), tetradeca(diaminobutyric acid), pentadeca(diaminobutyric acid), hexadeca(diaminobutyric acid), heptadeca(diaminobutyric acid), octadeca(diaminobutyric acid), nonadeca(diaminobutyric acid), lysine, dilysine, trilysine, tetralysine, pentalysine, hexalysine, heptalysine, octalysine, nonalysine, decalysine, undecalysine, dodecalysine, tridecalysine, tetradecalysine, pentadecalysine, hexadecalysine, heptadecalysine, octadecalysine, nonadecalysine, oligolysines, triornithine, tetraornithine, pentaornithine, hexaornithine, heptaornithine, octaornithine, nonaornithine, decaornithine, undecaornithine, dodecaornithine, tridecaornithine, tetradecaornithine, pentadecaornithine, hexadecaornithine, heptadecaornithine, octadecaornithine, nonadecaornithine, tridiaminobutyric acid, tetradiaminobutyric acid, pentadiaminobutyric acid, hexadiaminobutyric acid, heptadiaminobutyric acid, octadiaminobutyric acid, nonadiaminobutyric acid, decadiaminobutyric acid, undecadiaminobutyric acid, dodecadiaminobutyric acid, tridecadiaminobutyric acid, tetradecadiaminobutyric acid, pentadecadiaminobutyric acid, hexadecadiaminobutyric acid, heptadecadiaminobutyric acid, octadecadiaminobutyric acid, nonadecadiaminobutyric acid,
- or a polycarboxylate in bound form comprising at least 2 carboxylate groups (preferably further comprising a functional group, which is preferably an additional amino group or a carboxylic acid group, more preferably an additional carboxylic acid group),
   preferably selected from di(glutamic acid), tri(glutamic acid), tetra(glutamic acid), penta(glutamic acid), hexa(glutamic acid), hepta(glutamic acid), octa(glutamic acid), nona(glutamic acid), deca(glutamic acid), undeca(glutamic acid), dodeca(glutamic acid), trideca(glutamic acid), tetradeca(glutamic acid), pentadeca(glutamic acid), hexadeca(glutamic acid), heptadeca(glutamic acid), octadeca(glutamic acid), nonadeca(glutamic acid), di(aspartic acid), tri(aspartic acid), tetra(aspartic acid), penta(aspartic acid), hexa(aspartic acid), hepta(aspartic acid), octa(aspartic acid), nona(aspartic acid), deca(aspartic acid), undeca(aspartic acid), dodeca(aspartic acid), trideca(aspartic acid), tetradeca(aspartic acid), pentadeca(aspartic acid), hexadeca(aspartic acid), heptadeca(aspartic acid), octadeca(aspartic acid), nonadeca(aspartic acid), polyethyleneimines, and polyvinylamines.

In a preferred embodiment, a moiety Hyp^{y} of formula (III) comprises, preferably consists of a moiety selected from the group comprising, in particular consisting of, in bound form dilysine, trilysine, tetralysine, pentalysine, hexalysine, heptalysine, octalysine, nonalysine, decalysine, undecalysine, dodecalysine, tridecalysine, tetradecalysine, pentadecalysine, hexadecalysine, heptadecalysine, octadecalysine, nonadecalysine, triornithine, tetraornithine, pentaornithine, hexaornithine, heptaornithine, octaornithine, nonaornithine, decaornithine, undecaornithine, dodecaornithine, tridecaornithine, tetradecaornithine, pentadecaornithine, hexadecaornithine, heptadecaornithine, octadecaornithine, nonadecaornithine, tridiaminobutyric acid, tetradiaminobutyric acid, pentadiaminobutyric acid, hexadiaminobutyric acid, heptadiaminobutyric acid, octadiaminobutyric acid, nonadiaminobutyric acid, decadiaminobutyric acid, undecadiaminobutyric acid, dodecadiaminobutyric acid, tridecadiaminobutyric acid, tetradecadiaminobutyric acid, pentadecadiaminobutyric acid, hexadecadiaminobutyric acid, heptadecadiaminobutyric acid, octadecadiaminobutyric acid, nonadecadiaminobutyric acid, di(glutamic acid), tri(glutamic acid), tetra(glutamic acid), penta(glutamic acid), hexa(glutamic acid), hepta(glutamic acid), octa(glutamic acid), nona(glutamic acid), deca(glutamic acid), undeca(glutamic acid), dodeca(glutamic acid), trideca(glutamic acid), tetradeca(glutamic acid), pentadeca(glutamic acid), hexadeca(glutamic acid), heptadeca(glutamic acid), octadeca(glutamic acid), nonadeca(glutamic acid), di(aspartic acid), tri(aspartic acid), tetra(aspartic acid), penta(aspartic acid), hexa(aspartic acid), hepta(aspartic acid), octa(aspartic acid), nona(aspartic acid), deca(aspartic acid), undeca(aspartic acid), dodeca(aspartic acid), trideca(aspartic acid), tetradeca(aspartic acid), pentadeca(aspartic acid), hexadeca(aspartic acid), heptadeca(aspartic acid), octadeca(aspartic acid), nonadeca(aspartic acid), polyethyleneimines, and low-molecular weight PEI.

More preferably, a moiety Hyp^{y} of the carrier of formula (III) comprises, preferably consists of a moiety selected from the group comprising, more preferably consisting of, in bound form, trilysine, tetralysine, pentalysine, hexalysine, heptalysine, octalysine, nonalysine, decalysine, undecalysine, dodecalysine, tridecalysine, tetradecalysine, pentadecalysine, hexadecalysine, and heptadecalysine, even more preferably Hyp¹ and Hyp² are independently comprising, preferably consisting of, in bound form, trilysine, heptalysine or pentadecalysine.

In a preferred embodiment, Hyp^{y} of formula (III) has a molecular weight in the range of from 0.1 kDa to 4 kDa, more preferably 0.2 kDa to 2 kDa.

In a further preferred embodiment, a moiety Hyp^{y} of formula (III) has at least 1 branching and is conjugated to at least 2 moieties L of formula (Ia) and has at most 63 branchings and is at most conjugated to 64 moieties L of formula (Ia), more preferably each moiety Hyp^{y} of formula (III) has at least 1 branching and is conjugated to at least 2 moieties L of formula (Ia) and has at most 31 branchings and is at most conjugated to 32 moieties L of formula (Ia).

In a preferred embodiment, the carrier of formula (III) is characterized in that the carrier moiety comprises a quaternary carbon, in particular a quaternary carbon of a branching core moiety B, wherein B of formula (III) is pentarythritol in bound form. Preferably, each A of formula (III) is independently a PEG-based polymeric chain terminally attached to the quaternary carbon of pentaerythritol via the -CH₂-O- moieties of the branching core moiety pentaerythritol by a permanent covalent linkage, and the distal end of the PEG-based polymeric chain is covalently bound to a branched moiety Hyp^{y} of formula (III), each branched moiety Hyp^{y} of formula (III) is conjugated to reversible prodrug linker moieties L of formula (Ia).

In one preferred embodiment, a moiety Hyp^{y} of formula (III) comprises, preferably consists of branched polyamines comprising at least 2 amine groups. Preferably, the branched polyamine comprising at least 2 amine groups comprises one or more lysine residues in bound form. Preferably, each moiety Hyp^{y} of formula (III) has a molecular weight in the range of from 0.1 kDa to 4 kDa, particular 0.2 to 2 kDa. In a preferred embodiment, a carrier moiety B-(A-Hyp^{y})ₙ of formula (III), wherein n = 4, consists of the same or different moieties Hyp^{y} and that each Hyp^{y} can be chosen independently. In a preferred embodiment, all moieties Hyp_{y} of formula (III) are the same.

In a preferred embodiment, a moiety Hyp^{y} of formula (III) comprises, in particular consists of, between 1 and 32 lysines in bound form, preferably of 1, 3, 7 or 15 lysines in bound form, more preferably of 1, 3 or 7 lysines in bound form. Most preferably, Hyp^{y} of formula (III) comprises, in particular consists of heptalysinyl.

Preferably, the carrier moiety B(̵A-Hyp^{y})ₙ of formula (III), wherein n is preferably 4, has a molecular weight in the range of from 1 kDa to 80 kDa, more preferably 1 kDa to 40 kDa and even more preferably 10 kDa to 40 kDa.

Preferred carrier moieties B(̵A-Hyp^{y})₄ of formula (III) are selected from structures (i-y) to (iii-y): 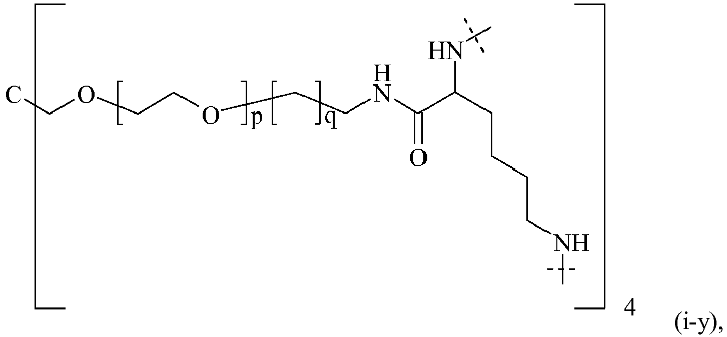 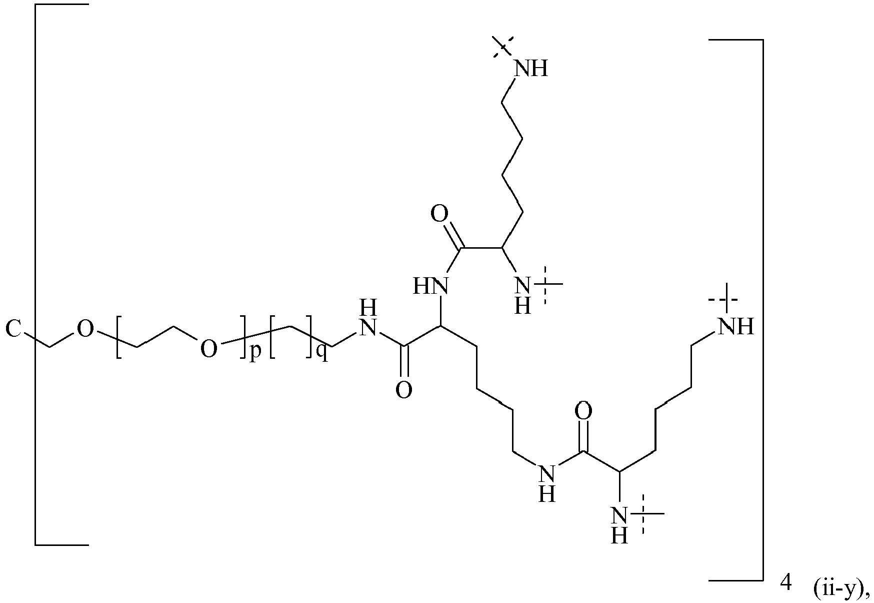 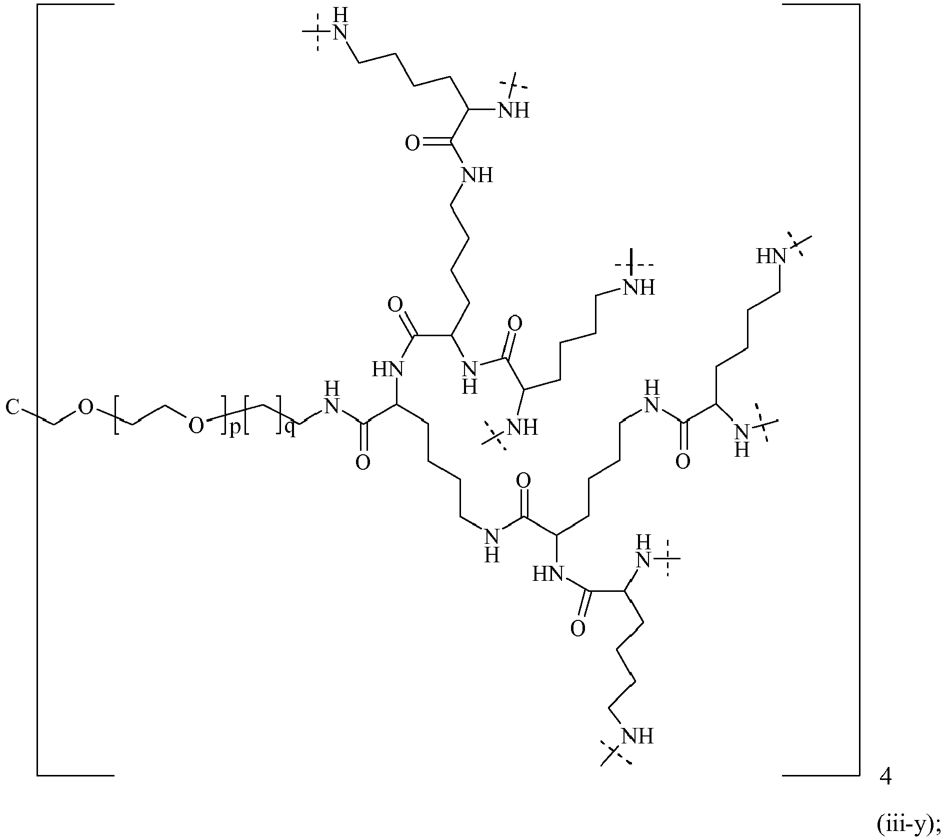 wherein
dashed lines indicate attachment to moieties L of formula (Ia),
p is an integer of from 5 to 2000, preferably from 10 to 1000, more preferably from 100 to 1000,
q is 1 or 2.

In a preferred embodiment, B of formula (III) is pentaerythritol.

In another preferred embodiment a carrier moiety POL of formula (Ia) and (Ib) is a protein carrier which comprises, in particular consists of an amino acid sequence of at least 100 amino acid residues.

In another preferred embodiment, a protein carrier POL of formula (Ia) and (Ib) is in random coil conformation.

In another preferred embodiment, the protein carrier POL of formula (Ia) and (Ib) comprises, in particular consists of alanine, serine and proline residues.

In the preferred embodiment, the protein carrier POL of formula (Ia) and (Ib) comprises, in particular consists of an amino acid sequence of at least 100 amino acid residues, and
wherein the amino acid sequence of at least 100 amino acid residues is in random coil conformation, and,
wherein the amino acid sequence of at least 100 amino acid residues comprises alanine, serine and proline residues.

Preferably, the protein carrier a protein carrier POL of formula (Ia) and (Ib) is composed of an amino acid sequence comprising at least about 100 amino acid residues, at least 100 amino acid residues, consisting of alanine, serine and proline residues which have a random coil conformation at physiological conditions. It is understood that the protein carrier POL of formula (Ia) and (Ib) may transiently or temporarily not form a random coil, for example when present in a lyophilisate or dried composition.

In one embodiment the protein carrier POL of formula (Ia) and (Ib) has a random coil conformation with an amino acid sequence of maximally about 3000 amino acid residues, preferably of maximally about 1500 amino acid residues, more preferably of maximally about 900 amino acid residues, even more preferably of maximally about 700 amino acid residues, particularly preferably of maximally about 600 amino acid residues. Thus, the amino acid sequence forming random coil conformation is maximally about 500 amino acid residues or of maximally about 450 amino acid residues in length.

Accordingly, the protein carrier POL of formula (Ia) and (Ib), in particular the amino acid sequence forming random coil conformation of the protein carrier POL of formula (Ia) and (Ib) is about 100 to about 3000 amino acid residues in length.

In particular embodiments said amino acid sequence forming random coil conformation of about 100 to 1000 amino acid residues is as characterized herein, i.e. comprising alanine, serine and proline as main or unique residues as defined below.

The protein carrier moiety POL of formula (Ia) and (Ib) consists mainly of the three amino acid residues alanine, serine and proline, and wherein all three amino acids are present in a protein carrier moiety POL of formula (Ia) and (Ib), whereby proline residues represent preferably about 4 % to about 40 % of the protein carrier POL of formula (Ia) and (Ib). The alanine and serine residues preferably comprise the remaining at least 60 % to 96 % of the protein carrier POL of formula (Ia) and (Ib). However, as will be detailed herein below said protein carrier POL of formula (Ia) and (Ib) may also comprise further amino acids differing from alanine, serine, and proline, i.e. as minor constituents.

The term "minor constituent" as used herein means that maximally 10 % (i.e. maximally 10 of 100 amino acids) may be different from alanine, serine and proline, preferably maximally 8 % (i.e. maximally 8 of 100 amino acids) may be different than alanine, serine and proline, more preferably maximally 6 % (i.e. maximally 6 of 100 amino acids) may be different from alanine, serine and proline, even more preferably maximally 5 % (i.e. maximally 5 of 100 amino acids) may be different from alanine, serine and proline, particularly preferably maximally 4 % (i.e. maximally 4 of 100 amino acids) may be different from alanine, serine and proline, more particularly preferably maximally 3 % (i.e. maximally 3 of 100 amino acids) may be different from alanine, serine and proline, even more particularly preferably maximally 2 % (i.e. maximally 2 of 100 amino acids) may be different from alanine, serine and proline and most preferably maximally 1 % (i.e. maximally 1 of 100 of the amino acids) that encode the protein carrier POL of formula (Ia), (Ib) and (Id) may be different from alanine, serine and proline. Said amino acids different from alanine, serine and proline may be selected from the group of natural or proteinogenic amino-acids consisting of Arg, Asn, Asp, Cys, Gln, Glu, Gly, His, Ile, Leu, Lys, Met, Phe, Thr, Trp, Tyr, and Val. Minor constituents may also be selected from non-naturally occurring amino acids, such as, for example, hydroxyproline or selenomethionine or other modified natural amino acids.

The term "at least about 100/150/200/250/300/300/350 (etc) amino acid residues" is not limited to the concise number of amino acid residues but also comprises amino acid stretches that comprise an additional 10 % to 20 % or comprise 10 % to 20 % less residues. For example "at least about 100 amino acid residues" may also comprise 80 to 100 and about 100 to 120 amino acid residues.

In one embodiment, the protein carrier POL of formula (Ia) and (Ib) comprises a plurality of polymer cassettes wherein said polymer cassettes consist of Ala, Ser, and/or Pro, and wherein no more than 6 consecutive amino acid residues of the polymer cassettes, preferably of the protein carrier POL of formula (Ia) and (Ib) are identical and wherein said proline residues constitute more than 4 % and less than 40 % of the amino acids of said protein carrier POL of formula (Ia) and (Ib).

In one embodiment, the protein carrier moiety POL of formula (Ia) and (Ib) comprises, preferably consists of a plurality of amino acid repeats,
wherein said repeats consist of Ala, Ser, and Pro residues,
and wherein no more than 6 consecutive amino acid residues of the carrier moiety POL of formula (Ia) and (Ib) are identical.

In a preferred embodiment, said proline residues constitute more than 4 % and less than 40 % of the amino acids of the protein carrier moiety POL of formula (Ia) and (Ib).

In a further preferred embodiment, the protein carrier moiety POL of formula (Ia) and (Ib) comprises, in particular consists of an amino acid sequence of about 100 to 3000 amino acid residues forming random coil conformation.

The protein carrier POL of formula (Ia) and (Ib) may comprise a plurality of identical polymer cassettes or a plurality of non-identical polymer cassettes. Non-limiting examples of polymer cassettes consisting of Ala, Ser and/or Pro residues are provided herein below; see SEQ ID NO:9, SEQ ID NO:10, SEQ ID NO:11, SEQ ID NO:12, SEQ ID NO:13 and SEQ ID NO:14 or peptide fragments or multimers of these sequences. A polymer cassette may consist of at least 3, 4, 5, 6, 7, 8, 9, 10, 11, 12, 13, 14, 15, 16, 17, 18, 19, 20, 21, 22, 23, 24, 25, 26, 27, 28, 29, 30 or more amino acid residues, wherein each polymer cassette comprises (an) Ala, Ser, and/or Pro residue(s), preferably (an) Ala, Ser, and Pro residue(s).

In one embodiment, the polymer cassette does not comprise more than 100 amino acid residues. Preferably, a polymer cassette as defined herein comprises more than about 4 %, preferably more than about 5 %, even more preferably more than about 6%, particularly preferably more than about 8 %, more particularly preferably more than about 10 %, even more particularly preferably more than about 15 % and most preferably more than about 20 % proline residues. Such polymer cassette as defined herein preferably comprises less than about 40 % or less than about 35 % proline residues.

In one embodiment the protein carrier POL of formula (Ia) and (Ib) is of formula (b):

Serₓ[Ala_{y}Ser_{z}]ᵥ (b),

which formula further comprises proline residues as defined herein and wherein
x is independently selected from integer 0 to 6,
each y is independently selected from integer ranging of from 1 to 6,
each z is independently selected from integer ranging of from 1 to 6.
v is any integer so that the protein carrier POL of formula (Ia) and (Ib) consists of at least about 100 amino acid residues, and in particular of at least about 100 to about 3000 amino acid residues, preferably to about 2000 and more preferably to about 1000 amino acid residues.

In one embodiment, all y of formula (b) and z of formula (b) of the v Ala_{y} Ser_{z} monomer moieties of formula (b) are identical. In another embodiment, the y of formula (b) and z of formula (b) of the v Ala_{y} Ser_{z} monomer moieties of formula (b) are different.

In preferred embodiments, the protein carrier POL of formula (Ia) and (Ib) comprises no more than 5 identical consecutive amino acid residues, more preferably no more than 4 identical consecutive amino acid residues and most preferably no more than 3 identical consecutive amino acid residues.

As already indicated herein above, the protein carrier POL of formula (Ia) and (Ib) comprises proline residues, wherein said proline residues constitute more than about 4 %, preferably more than about 5 %, even more preferably more than about 6 %, particularly preferably more than about 8 %, more particularly preferably more than about 10 %, even more particularly preferably more than about 15 % and most preferably more than about 20 % of the amino acids constituting the protein carrier POL of formula (Ia) and (Ib). Such proline residues may be introduced at any position in formula (b). Preferably, the proline residues may be present in one or more of the v Ala_{y} Ser_{z} monomers of formula (b), and they may be present at the same or at different positions.

In another preferred embodiment, the protein carrier POL of formula (Ia) and (Ib) comprises more than about 4 % but less than about 50 %, preferably more than about 10 % but less than about 50 % and most preferably more than about 20 % but less than about 50 % alanine residues of the amino acids constituting the protein carrier POL of formula (Ia) and (Ib).

In a further preferred embodiment, the protein carrier POL of formula (Ia) and (Ib) comprises more than about 4 % and less than about 50 %, preferably more than about 10 % but less than about 50 % and most preferably more than about 20 % but less than about 50 % serine residues of the amino acids constituting the protein carrier POL of formula (Ia) and (Ib).

Accordingly, the protein carrier POL of formula (Ia) and (Ib) comprises about 35 % proline residues, about 50 % alanine residues and about 15 % serine residues of the amino acids constituting the protein carrier POL of formula (Ia) and (Ib). Alternatively, the protein carrier POL of formula (Ia) and (Ib) may comprise about 35 % proline residues, about 15 % alanine residues and about 50 % serine residues of the amino acids constituting the protein carrier POL of formula (Ia) and (Ib).

Preferably, the protein carrier POL of formula (Ia) and (Ib) is comprises one or more of the following alanine-serine polymer cassettes:
SEQ ID NO:1
   AAAASSASSASSSSSAAASA
SEQ ID NO:2
   AASAAASSAAASAAAASASS
SEQ ID NO:3
   ASASASASASASSAASAASA
SEQ ID NO:4
   SAASSSASSSSAASSASAAA
SEQ ID NO:5
   SSSSAASAASAAAAASSSAS
SEQ ID NO:6
   SSASSSAASSSASSSSASAA
SEQ ID NO:7
   SASASASASASAASSASSAS
SEQ ID NO:8
   ASSAAASAAAASSAASASSS
provided that the protein carrier POL of formula (Ia) and (Ib) further comprises proline residues as described herein.

The multimers of these alanine-serine polymer cassettes may form random coil conformation in case the resulting amino acid sequence further comprises proline residues as defined herein above.

In a preferred embodiment, the protein carrier POL of formula (Ia) and (Ib) comprises, preferably consists of one or more of the following polymer cassettes:
SEQ ID NO:9
   ASPAAPAPASPAAPAPSAPA
SEQ ID NO: 10
   AAPASPAPAAPSAPAPAAPS
SEQ ID No:1 1
   APSSPSPSAPSSPSPASPSS
   and
SEQ ID NO:15
   SAPSSPSPSAPSSPSPASPS.

SEQ ID NO:15 corresponds to the herein provided SEQ ID No:11 in a circularly permuted form, wherein the last serine was removed and another serine was appended as starting amino acid. As a consequence, multimers of this modified sequence possess essentially the same internal repeating unit as multimers of the non-modified sequence, except for the very first and the very last residue. Accordingly, SEQ ID NO:15 may be considered as an example of a further polymer cassette for the protein carrier POL of formula (Ia) and (Ib). It is clear for the person skilled in the art that also other polymer cassettes and (shorter) peptide fragments or circularly permuted versions of the herein provided amino acid polymers may be used as polymer cassettes for the protein carrier POL of formula (Ia) and (Ib).

Yet, even further and illustrative amino acid polymers forming random coil conformation may comprise amino acid sequences that may be selected from the group consisting of:
SEQ ID NO:12
   SSPSAPSPSSPASPSPSSPA,
SEQ ID NO:13
   AASPAAPSAPPAAASPAAPSAPPA, and
SEQ ID NO:14
   ASAAAPAAASAAASAPSAAA.

Therefore, preferred polymer cassettes for POL of formula (Ia) and (Ib) are selected from the following sequences:
ASPAAPAPASPAAPAPSAPA (SEQ ID NO:9),
AAPASPAPAAPSAPAPAAPS (SEQ ID NO:10),
APSSPSPSAPSSPSPASPSS (SEQ ID NO:11),
SSPSAPSPSSPASPSPSSPA (SEQ ID NO:12),
AASPAAPSAPPAAASPAAPSAPPA (SEQ ID NO:13), and
ASAAAPAAASAAASAPSAAA (SEQ ID NO:14);
or circular permuted versions or (a) multimer(s) of these sequences as a whole or parts of these sequences.

In one embodiment, the protein carrier moiety POL of formula (Ia) and (Ib) comprises at least one amino acid sequence selected from the group consisting of:
ASPAAPAPASPAAPAPSAPA (SEQ ID NO:9),
AAPASPAPAAPSAPAPAAPS (SEQ ID NO:10),
APSSPSPSAPSSPSPASPSS (SEQ ID NO:11),
SSPSAPSPSSPASPSPSSPA (SEQ ID NO:12),
AASPAAPSAPPAAASPAAPSAPPA (SEQ ID NO:13), and
ASAAAPAAASAAASAPSAAA (SEQ ID NO:14);
and circular permuted versions or (a) multimer(s) of these sequences as a whole or parts of these sequences.

Again, also (a) peptide fragment(s) or (a) multimer(s) or circularly permuted versions of these sequences and the sequences provided herein above may be employed as polymer cassettes for the protein carrier POL of formula (Ia) and (Ib).

Accordingly, the exemplified polymer cassettes may also provide for individual peptide fragments which may be newly combined to form further polymer cassettes.

In accordance with the above, the protein carrier POL of formula (Ia) and (Ib) may comprise a multimer consisting of either one of the amino acid sequences with SEQ ID NO:9, 10, 11, 12, 13 or 14 as disclosed herein above or may comprise a multimer consisting of more than one of amino acid sequences SEQ ID NO:9, 10, 11, 12, 13 and 14. Furthermore, it is envisaged that also peptide fragments or circularly permuted versions of these exemplified sequences may be used to build up further polymer cassettes of the protein carrier POL of formula (Ia) and (Ib).

In another embodiment, the protein carrier POL of formula (Ia) and (Ib) may comprise a multimer comprising, preferably consisting of a (circular) permutation of the amino acid sequence selected from the group consisting of SEQ ID NOs:9, 10, 11, 12, 13, 14, 15 and (a) multimers(s) of these (circular) permutated sequences.

In yet another embodiment, the protein carrier POL of formula (Ia) and (Ib) may comprise, preferably consist of a multimer consisting of a peptide fragment/part of the amino acid sequence selected from the group consisting of SEQ ID NO: 9, 10, 12, 13, 14, 15 and (a) multimers(s) of these exemplified polymer cassettes.

Peptide fragments of these sequences to be employed for the generation of the protein carrier POL of formula (Ia) and (Ib) may consist of at least 3, preferably of at least 4, more preferably of at least 5, even more preferably of at least 6, still more preferably of at least 8, particularly preferably of at least 10, more particularly preferably of at least 12, even more particularly preferably of at least 14, preferably of at least 6, still more preferably of at least 8, particularly preferably of at least 10, more particularly preferably of at least 12, even more particularly preferably of at least 14, even more particularly preferably of at least 16, and most preferably of at least 18 consecutive amino acids of the amino acid sequence selected from the group consisting of said SEQ ID NOs: 9, 10, 11, 12, 13 and 14.

For example, individual peptide fragments of the polymer cassettes may be combined to further individual polymer cassettes as long as the above-identified rules for the overall distribution and amount of alanine, serine and proline are respected. Again, these polymer cassettes may also comprise further amino acid residues, however only as minimal or minor constituents, i. e. maximally 10 %, preferably maximally 2 % of the individual polymer cassette. Said individual polymer cassettes consist of at least about 100 amino acid residues. Individual polymer cassettes may be combined in order to form longer random coil forming amino acid polymers, whereby a maximal length of the protein carrier POL of formula (Ia) and (Ib) is about 3000 amino acids. A preferred minor constituent of the protein carrier POL of formula (Ia) and (Ib) is lysine.

Preferably, Q in formula (Ic) and (Id) is selected from COOR⁹; OR⁹; C(O)R⁹; C(O)N(R⁹R^{9a}); S(O)₂N(R⁹R^{9a}); S(O)N(R⁹R^{9a}); S(O)₂R⁹; S(O)R⁹; N(R⁹)S(O)₂N(R^{9a}R^{9b}); SR⁹; N(R⁹R^{9a}); OC(O)R⁹; N(R⁹)C(O)R^{9a}; N(R⁹)S(O)₂R^{9a}; N(R⁹)S(O)R^{9a}; N(R⁹)C(O)OR^{9a}; N(R⁹)C(O)N(R^{9a}R^{9b}), OC(O)N(R⁹R^{9a}); T; C₁₋₅₀ alkyl; C₂₋₅₀ alkenyl; and C₂₋₅₀ alkynyl,
wherein T, C₁₋₅₀ alkyl, C₂₋₅₀ alkenyl, and C₂₋₅₀ alkynyl are optionally substituted with one or more R¹⁰, which are the same or different,
and wherein C₁₋₅₀ alkyl; C₂₋₅₀ alkenyl; and C₂₋₅₀ alkynyl are optionally interrupted by one or more groups selected from the group consisting of -T-, -C(O)O-; -O-; -C(O)-; -C(O)N(R¹¹)-; -S(O)₂N(R¹¹)-; -S(O)N(R¹¹)-; -S(O)₂-; -S(O)-; -N(R¹¹)S(O)₂N(R^{11a})-; -S-; -N(R¹¹)-; -OC(O)R¹¹; -N(R¹¹)C(O)-; -N(R¹¹)S(O)₂-; -N(R¹¹)S(O)-; -N(R¹¹)C(O)O-; -N(R¹¹)C(O)N(R^{11a})-; and -OC(O)N(R¹¹R^{11a}),
R⁹, R^{9a}, R^{9b} are independently selected from the group consisting of H; T; and C₁₋₅₀ alkyl; C₂₋₅₀ alkenyl; and C₂₋₅₀ alkynyl,
wherein T, C₁₋₅₀ alkyl, C₂₋₅₀ alkenyl, and C₂₋₅₀ alkynyl are optionally substituted with one or more R¹⁰, which are the same or different,
and wherein C₁₋₅₀ alkyl; C₂₋₅₀ alkenyl; and C₂₋₅₀ alkynyl are optionally interrupted by one or more groups selected from the group consisting of T, -C(O)O-; -O-; -C(O)-; -C(O)N(R¹¹)-; -S(O)₂N(R¹¹)-; -S(O)N(R¹¹)-; -S(O)₂-; -S(O)-; -N(R¹¹)S(O)₂N(R^{11a})-; -S-; -N(R¹¹)-; -OC(O)R¹¹; -N(R¹¹)C(O)-; -N(R¹¹)S(O)₂-; -N(R¹¹)S(O)-; -N(R¹¹)C(O)O-; -N(R¹¹)C(O)N(R^{11a})-; and -OC(O)N(R¹¹R^{11a});
T is selected from the group consisting of phenyl; naphthyl; indenyl; indanyl; tetralinyl; C₃₋₁₀ cycloalkyl; 4- to 7-membered heterocyclyl; and 9- to 11-membered heterobicyclyl, wherein T is optionally substituted with one or more R¹⁰, which are the same or different;
R¹⁰ is halogen; CN; oxo (=O); COOR¹²; OR¹²; C(O)R¹²; C(O)N(R¹²R^{12a}); S(O)₂N(R¹²R^{12a}); S(O)N(R¹²R^{12a}); S(O)₂R¹²; S(O)R¹²; N(R¹²)S(O)₂N(R^{12a}R^{12b}); SR¹²; N(R¹²R^{12a}); NO₂; OC(O)R¹²; N(R¹²)C(O)R^{12a}; N(R¹²)S(O)₂R^{12a}; N(R¹²)S(O)R^{12a}; N(R¹²)C(O)OR^{12a}; N(R¹²)C(O)N(R^{12a}R^{12b}); OC(O)N(R¹²R^{12a}); or C₁₋₆ alkyl, wherein C₁₋₆ alkyl is optionally substituted with one or more halogen, which are the same or different;
R¹¹, R^{11a}, R¹², R^{12a}, R^{12b} are independently selected from the group consisting of H; and C₁₋₆ alkyl, wherein C₁₋₆ alkyl is optionally substituted with one or more halogen, which are the same or different.

Preferably, a moiety L of formula (1a) or (1b) of the carrier-linked prodrugs of the present invention is selected from the following structures: 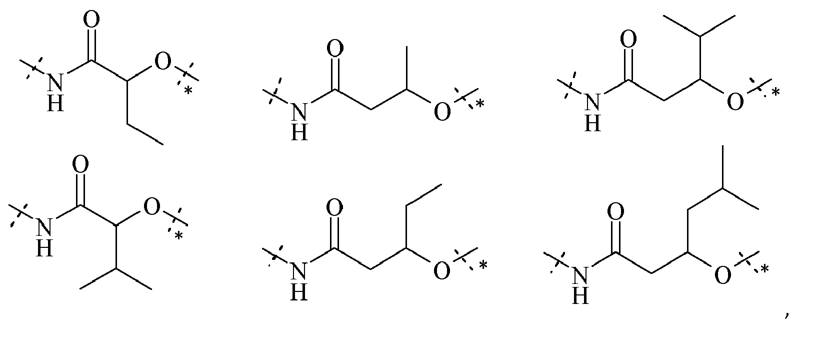 wherein dashed lines with an asterisk indicate attachment to a moiety D and unmarked dashed lines indicate attachment to the rest of the molecule.

The carrier-linked prodrug of the present invention comprises a biologically active moiety which comprises in free form at least one carboxyl group and which biologically active moiety may preferably be selected from the group of peptides, polypeptides, proteins, or small molecule biologically active moieties. The carrier-linked prodrug of the present invention comprises at least one moiety D which moiety D is attached to the reversible prodrug L via an ester group formed from the at least one carboxylic acid group of D. It is understood that the biologically active moiety may in addition to the at least one carboxyl group comprise one or more additional functional group(s), such as, for example, one or more amine, hydroxyl, phosphate and/or mercapto group(s).

Preferably, suitable peptide, polypeptide, protein, and protein mixture drugs for the carrier-linked prodrug of the present invention may be selected from the group consisting of ACTH, adenosine deaminase, agalsidase, albumin, alfa-1 antitrypsin (AAT), alfa-1 proteinase inhibitor (API), alglucosidase, alteplase, anistreplase, ancrod serine protease, antibodies (monoclonal or polyclonal and fragments or fusions), antithrombin III, antitrypsins, aprotinin, asparaginases, biphalin, bone-morphogenic proteins, calcitonin (salmon), collagenase, DNase, endorphins, enfuvirtide, enkephalins, erythropoietins, factor VIIa, factor VIII, factor VIIIa, factor IX, fibrinolysin, fusion proteins, follicle-stimulating hormones, granulocyte colony stimulating factor (G-CSF), galactosidase, glucagon, glucagon-like peptides like GLP-1, glucocerebrosidase, granulocyte macrophage colony stimulating factor (GM-CSF), chorionic gonadotropin (hCG), hemoglobins, hepatitis B vaccines, hirudin, hyaluronidases, idurnonidase, immune globulins, influenza vaccines, interleukines (1 alfa, 1 beta, 2, 3, 4, 6, 10, 11, 12), IL-1 receptor antagonist (rhIL-1ra), insulins, interferons (alfa 2a, alfa 2b, alfa 2c, beta 1a, beta 1b, gamma 1a, gamma 1b), keratinocyte growth factor (KGF), lactase, leuprolide, levothyroxine, luteinizing hormone, lyme vaccine, natriuretic peptide, pancrelipase, papain, parathyroid hormone, PDGF, pepsin, phospholipase-activating protein (PLAP), platelet activating factor alcetylhydrolase (PAF-AH), prolactin, protein C, octreotide, secretin, sermorelin, superoxide dismutase (SOD), somatropins (growth hormone), somatostatin, streptokinase, sucrase, tetanus toxin fragment, tilactase, thrombins, thymosin, thyroid stimulating hormone, thyrothropin, transforming growth factors, tumor necrosis factor (TNF), TNF receptor-IgG Fc, tissue plasminogen activator (tPA), transferrin, TSH, urate oxidase, urokinase, Fab (fragment, antigen-binding), F(ab)2 fragments, Fc (fragment, crystallizable), pFc' fragment, Fv (fragment, variable), scFv (single-chain variable fragment), di-scFv/diabodies, bi-specific T-cell engager, CDRs (complementarity determining regions), single-domain antibodies (sdABs/Nanobodies), heavy chains (α, δ, ε, γ, µ) or heavy chain fragments, light chains (λ, κ) or light chain fragments, VH fragments (variable region of the heavy chain), VL fragments (variable region of the light chain), VHH fragments, VNAR fragments, shark-derived antibody fragments and affinity scaffold proteins, Kunitz domain-derived affinity scaffold proteins, centyrin-derived affinity scaffold proteins, ubiquitin-derived affinity scaffold proteins, lipocalin-derived affinity scaffold proteins, ankyrin-derived affinity scaffold proteins, Versabodies (disulfide-rich affinity scaffold proteins), fibronectin-derived affinity scaffold proteins, cameloid-derived antibody fragments and affinity scaffold proteins, llama-derived antibody fragments and affinity scaffold proteins, transferrin-derived affinity scaffold proteins, and Squash-type protease inhibitors with cysteine-knot scaffold-derived affinity scaffold proteins.

Attachment of carboxyl group comprising peptide, polypeptide and protein biologically active moieties to the reversible prodrug linker moiety of formula (Ia) or (Ib) occurs either through the C-terminal carboxyl group or through a carboxyl group of an amino acid side chain of said polypeptide and protein biologically active moieties, such as through the carboxyl group of a glutamic acid or aspartic acid residue.

Preferably, suitable small molecule drugs for the carrier-linked prodrugs of the present invention comprising a carboxyl group are selected from the list consisting of (-)-Subersic acid, (+)-Deoxoartelinic acid, (+)-Hemipalmitoylcarnitinium, (+)-Indobufen, (+)-SCH-351448, (E)-p-Coumaroylquinic acid, (Z)-Indenaprost, [111In-DTPA-Pro1,Tyr4]bombesin, [90Y]-DOTAGA-substance P, [psi[CH2NH]Tpg4]Vancomycin aglycon, 111In-Pentetreotide, 11-Keto-Beta-Boswellic Acid, 15-Methoxypinusolidic acid, 1-Methyl-D-tryptophan, 3,5-Dicaffeoylquinic acid, 3-MATIDA, 3-O-Acetyloleanolic acid, 4-Aminosalicylic acid, 6alpha-Fluoroursodeoxycholic acid, 6-Carboxygenistein, 7-Chlorokynurenic acid, 8-Carboxy-isoiantheran A, 99mTc-c(RGDfK)2HYNIC, A-42867 pseudoaglycone, Aceclofenac, Acemetacin, Aceneuramic acid sodium salt, Acetyl-11-Keto-Beta-Boswellic Acid, Acetyl-Beta-Boswellic Acid, Acetylcysteine, Achimillic Acids, Acipimox, Acitazanolast, Acrivastine, Actarit, Adapalene, Adarotene, Ademetionine tosylate sulfate, Adxanthromycin A, Ajulemic acid, Alacepril, Aladapcin, Aleglitazar, Alitretinoin, Alminoprofen, Alogliptin benzoate, alpha-Linolenic acid, alpha-Lipoic acid, alpha-Methyltryptophan, Alprostadil, Altemicidin, Alutacenoic acid B, Alvimopan hydrate, Amiglumide, Amineptine, Aminocaproic acid, Aminolevulinic acid hydrochloride, Amlexanox, Amoxicillin trihydrate, Amphotericin B, Amsilarotene, Anakinra, Antiflammin-1, Antiflammin-2, Antiflammin-3, Apalcillin sodium, Aplaviroc hydrochloride, Argatroban monohydrate, Argimesna, Artelinate, Artepillin C, Artesunate, Arundifungin, Ascosteroside, Asiatic acid, Aspirin, Aspoxicillin, Assamicin I, Assamicin II, Ataluren, Atorvastatin, Atorvastatin calcium, Atrasentan, Azaromycin SC, Azelaic Acid, Azepinostatin, Azilsartan, Azoxybacilin, Aztreonam, Aztreonam L-lysine, Azumamide E, Baclofen, Bafilomycin C1, Baicalin, Balhimycin, Balofloxacin, Balofloxacin dihydrate, Balsalazide disodium, Bamirastine hydrate, Belactosin A, Belactosin C, Benanomicin A, Benanomicin B, Benastatin A, Benastatin B, Benazepril hydrochloride, Benthocyanin A, Bepotastine besilate, Beraprost sodium, Besifloxacin hydrochloride, Beta-Boswellic Acid, beta-Hydroxy beta-methylbutyrate, Betamipron, Beta-Sialosylcholesterol Sodium Salt, Bevirimat, Bexarotene, Bezafibrate, Biapenem, Bilastine, Bimosiamose, Bindarit, Binfloxacin, Biphenyl-indanone A, Boc-Belactosin A, Borrelidin, Brasilicardin A, Brasilinolide A, Bremelanotide, Brevifolin carboxylic acid, Bucillamine, Bumetanide, Bungeolic acid, Buprenorphine hemiadipate, Buprenorphine-Val-carbamate, Butibufen, Butoctamide hemisuccinate, Butyzamide, Cabin 1, Cadrofloxacin hydrochloride, Calbistrin A, Calbistrin B, Calbistrin C, Calbistrin D, Calcium-like peptide 1, Calcium-like peptide 2, Caloporoside B, Caloporoside C, Caloporoside D, Caloporoside E, Caloporoside F, Calpinactam, Calteridol calcium, Camprofen, Candesartan, Candoxatril, Candoxatrilat, Canfosfamide hydrochloride, Canrenoate potassium, Caprazamycin A, Caprazamycin B, Caprazamycin C, Caprazamycin E, Caprazamycin F, Captopril, Carbidopa, Carmoxirole hydrochloride, Carprofen, Cefaclor, Cefalexin monohydrate, Cefbuperazone sodium, Cefcanel, Cefdaloxime, Cefdinir, Cefetecol, Cefixime, Cefmatilen hydrochloride hydrate, Cefmenoxime hydrochloride, Cefminox sodium, Cefodizime, Cefonicid sodium, Cefoperazone sodium, Cefoselis sulfate, Cefotiam hydrochloride, Cefoxitin, Cefpimizole sodium, Cefpiramide sodium, Cefprozil, Cefprozil monohydrate, Ceftaroline fosamil acetate, Ceftazidime, Ceftibuten, Ceftobiprole, Cefuroxime, Ceranapril, Cerivastatin sodium, Ceruletide diethylamine, Cetefloxacin, Cetirizine hydrochloride, Chenodeoxycholic acid, Chinoin-169, Chlorambucil, Chloroorienticin A, Chloroorienticin B, Choline fenofibrate, Choline thioctate, Chrolactomycin, Cilastatin sodium, Cilazapril, Cilengitide, Cilomilast, Ciluprevir, Cinaciguat, Cinalukast, Cinatrin A, Cinatrin B, Cinatrin C1, Cinatrin C2, Cinatrin C3, Cinnatriacetin A, Cinnatriacetin B, Ciprofibrate, Ciprofloxacin hydrochloride, Circinamide, Cispentacin, Citrullimycine A, Clavaric acid, Clavulanate potassium, Clinofibrate, Clopidogrel Sulfate, Colletoic acid, Complestatin, Conagenin, Cosalane, Creatine phosphate, Cyclocreatine, Cycloplatam, Cyclothialidine, Cytomodulin, Cytosporic acid, Dabigatran, Daglutril, Dalargin, Dalbavancin, Danegaptide hydrochloride, Danofloxacin, Darinaparsin, Darusentan, Daurichromenic acid, Davunetide, Decahydromoenomycin A, Decaplanin, Decatromicin A, Decatromicin B, Deferasirox, Delafloxacin, Delapril Hydrochloride, Deltibant, Deoxylaidlomycin, Deoxynegamycin, Dersalazine, Desacetylvinblastinehydrazide/folate conjugate, Desferri-danoxamine, Desferrinordanoxamine, Desglugastrin tromethamine, Desmin-370, Dexibuprofen, Dexibuprofen lysine, Dexketoprofen, Dexketoprofen choline, Dexketoprofen D,L-lysine, Dexketoprofen lysine, Dexketoprofen meglumine, Dexketoprofen trometamol, Dexloxiglumide, Dexpemedolac, dextro-Ciprofibrate, Dexylosylbenanomycin A, Diacerein, Diazaphilonic acid, Di-Calciphor, Difenoxin, Diflunisal, Dihydroavenanthramide D, Dihydrogranaticin B, Dihydroisosteviol, Dihydrolipoic acid, Disalazine, Disila-bexarotene, Disodium cromproxate, Disodium lettusate, Doqualast, Doripenem, Dormitroban, Dorrigocin A, Dorrigocin B, Droxidopa, DTPA-adenosylcobalamin, Duramycin, Dynemicin A, Ecabet Sodium, Ecenofloxacin hydrochloride, Econazole Sulfosalicylate, Edetic acid, Edotreotide yttrium, Efletirizine, Eflornithine hydrochloride, Eglumetad hydrate, Elansolid C1, Elarofiban, Elastatinal B, Elastatinal C, Elsibucol, Eltrombopag olamine, Elvitegravir, Emricasan, Enalapril maleate, Enalapril nitrate, Enalaprilat, Enfumafungin, Enkastin (D), Enkastin AD, Enkastin AE, Enkastin ID, Enkastin IE, Enkastin VD, Enkastin VE, Enoloxone, Enoxacin, Enrasentan, Enrofloxacin, Epalrestat, Epidioxymanadic acid A, Epidioxymanadic acid B, Epithalon, Epofolate, Epoprostenol sodium, Epostatin, Epristeride, Eprosartan mesilate, Eprotirome, Eptaloprost, Eptastatin sodium, Eptastigmine Tartrate, Eptifibatide, Erdosteine, Eremomycin, Ertapenem sodium, Ertiprotafib, Eryloside F, Esafloxacin Hydrochloride, Esonarimod, Etacrynic acid, Etalocib sodium, Etodolac, Etretin, Evatanepag, Evernimicin, Exisulind, Ezetimibe glucuronide, Fandofloxacin hydrochloride, Faranoxi, Farglitazar, Faropenem sodium, Fasobegron hydrochloride, Febuxostat, Feglymycin, Felbinac, Felbinac Lysine Salt, Fenbufen, Fexofenadine hydrochloride, Fidexaban, Finafloxacin hydrochloride, Fleroxacin, Flobufen, Flomoxef Sodium, Flunoprost, Flunoxaprofen, Flurbiprofen, Fluvastatin sodium, Folinic acid, Fondaparinux sodium, Fosfosal, Fradafiban, Frusemide, Fudosteine, Furprofen, G1 peptide, Gabadur, Gabapentin, Gabapentin enacarbil, Gabusectin, Gadobenic acid dimeglumine salt, Gadobutrol, Gadocoletic acid trisodium salt, Gadodenterate, Gadomelitol, Gadopentetate dimeglumine, Gadoterate meglumine, Gadoteridol, Gambogic acid, Gamendazole, Gamma-Linolenic Acid, Ganefromycin Alpha, Ganefromycin Beta, Ganglioside GM1, Ganoderic acid X, Garenoxacin mesilate, Gastrazole, Gatifloxacin, Gemfibrozil, Gemifloxacin mesilate, Gemopatrilat, Gilatide, Gimatecan, Giripladib, Glaspimod, Glucarolactam potassium, Gludopa, Glutathione Monoethyl Ester, Glutathione Monoisopropyl Ester, Glycine-proline-Melphalan, Glycopin, Glycyrrhizinic acid, Golotimod, Goodyeroside B, Goralatide, Grepafloxacin hydrochloride, GS-143, Haterumadioxin A, Haterumadioxin B, Helvecardin A, Helvecardin B, Heptelidic acid chlorohydrin, Hericenal A, Hericenal B, Hericenal C, Homoindanomycin, Hongoquercin A, Hongoquercin B, Human angiotensin II, Hyaluronate sodium, Hydrostatin A, Ibuprofen, Icatibant acetate, Icofungipen, Idrapril, Ifetroban, Ilepatril, Iloprost, Imidapril, Imidapril hydrochloride, Imiglitazar, Imipenem, Indanaprost (S), Indanomycin, Indeglitazar, Indobufen, Indole-3-propionic acid, Indometacin, Indomethacin trometamol, Indoxam, Indynaprost, Inogatran, Inosiplex, Iododiflunisal, Iodofiltic acid-[1231], Iodostearic Acid, Iralukast, Iralukast sodium, Isalsteine, Isobongkrekic acid, Isotretinoin, Itavastatin calcium, Itriglumide, Kaitocephalin, Kanglemycin A, Kapurimycin A1, Kapurimycin A3, Ketoprofen, Ketoprofen lysine, Ketorolac, Ketorolac tromethamine, Khafrefungin, Kijimicin, Kistamicin A, L-4-Oxalysine, Labradimil, Lamectacin, Lamifiban, Lanthiopeptin, Lapaquistat acetate, Larazotide acetate, Laropiprant, Latamoxef sodium, L-Chicoric acid, Lenapenem hydrochloride, Lenapenem hydrochloride hydrate, Levocabastine hydrochloride, Levocetirizine dihydrochloride, levo-Ciprofibrate, Levodopa, Levodopa 3-O-glucoside, Levodopa 4-O-glucoside, Levofloxacin, Levonadifloxacin arginine salt, L-Homothiocitrulline, Licofelone, Licorice-saponin C2, Lidorestat, Limaprost alfadex, Limazocic, Linoleic acid 18:2w6-cis,9-cis, Linotroban, Lintitript, Lipohexin, Lisinopril, Lithium succinate, Lithospermic acid B magnesium salt, Loloatin B, Lomefloxacin hydrochloride, Lometrexol, Longestin, Lonidamine, Loracarbef hydrate, Lorglumide, Lotrafiban, Loxiglumide, L-Simexonyl homocysteine, L-Thiocitrulline, Lubiprostone, Lumiracoxib, Lu-Tex bis(gluconate), Lysinated-betulonic acid, Lysine acetylsalicylate, Macrocarpin B, Madecassic acid, Maracenin A1, Maracenin A2, Maracenin B1, Maracenin B2, Maracenin C1, Maracenin C2, Maracenin D1, Maracenin D2, Marbofloxacin, Maslinic acid, Matristatin A1, Matristatin A2, Matteuorienate A, Matteuorienate B, Matteuorienate C, Mebrofenin, Meclinertant, Mefenamic acid, Melagatran, Memno-peptide A, Meptazinol-Val-carbamate, Meropenem, Mersacidin, Mesalazine, Metesind glucuronate, Methanobactin, Methotrexate, Methoxatin, Methyldopa, Methylenolactocin, Methylhomoindanomycin, Metiapril, Metirosine, Micacocidin A, Micacocidin B, Midafotel, Midoriamin, Milrinone Lactate, Minerval, Mipitroban, Mispyric acid, Mixanpril, Moenomycin A chloride bismuth salt, Moexipril hydrochloride, Moexiprilat, Mofezolac, Momordin Ic, Monamidocin, Monoethanolamine oleate, Montelukast sodium, Morphine Glucuronide, Moxifloxacin hydrochloride, Mumbaistatin, Mupirocin, Muraglitazar, Muraminomicin A, Muraminomicin B, Muraminomicin C, Muraminomicin D, Muraminomicin E1, Muraminomicin E2, Muraminomicin F, Muraminomicin G, Muraminomicin H, Muraminomicin I, Muraminomicin Z1, Muraminomicin Z2, Muraminomicin Z3, Muraminomicin Z4, Mureidomycin A, Mureidomycin B, Mureidomycin C, Mureidomycin D, Mureidomycin E, Mureidomycin F, Mureidomycins, Mycaperoxide A, Mycaperoxide B, Mycestericin E, Mycophenolic acid sodium salt, Myriceric acid A, Mytolbilin acid, Nadifloxacin, Nafagrel hydrochloride, Nafagrel hydrochloride hemihydrate, Nagstatin, Napirimus, Napsagatran, Napsamycin A, Napsamycin B, Napsamycin C, Napsamycin D, Nateglinide, Naveglitazar, Nebostinel, Nemonoxacin, Neu5Ac2en, Niacin, Niglizin, Nileprost beta-cyclodextrin clathrate, Nooglutil, Norfloxacin, Norfloxacin succinil, Obeticholic acid, Octacosamicin A, Octacosamicin B, O-Demethylchlorothricin, Ofloxacin, Olamufloxacin, Olamufloxacin mesilate, Olanzapine pamoate, Oleanolic acid, Olmesartan, Olopatadine Hydrochloride, Olsalazine sodium, Omapatrilat, Onnamide A, OPC-17083, Opiorphin, Orbifloxacin, Oreganic acid, Orienticin A, Orienticin B, Orienticin C, Orienticin D, Oritavancin, Orniplabin, Oseltamivir carboxylate, Ovothiol A, Ovothiol B, Ovothiol C, Oxaprozin, Oxeglitazar, Oxiglutatione sodium, Oxymorphone-Val-carbamate, Oxynor, Ozagrel hydrochloride, Ozenoxacin, Pactimibe, Padoporfin, Paeciloquinone B, Paeciloquinone D, Paldimycin B, Palovarotene, Panipenem, Parasin I, Parinaric acid, Paulomycin, Paulomycin A2, Paulomycin B, Paulomycin C, Paulomycin D, Paulomycin E, Paulomycin F, Pazufloxacin, Pazufloxacin mesilate, Pefloxacin, PEG-vancomycin, Pelagiomicin C, Peliglitazar, Pelitrexol, Pelretin, Penasterol, Penicillamine, Peramivir, Perindopril, PG-camptothecin, Phomallenic acid C, Phomoidride A, Phomoidride B, Phosphinic cyclocreatine, Phosphosalsalate, Physostigmine salicylate, Pibaxizine, Pidotimod, Piraxostat, Piretanide, Pirfenoxone, Pirprofen, Pivagabine, Pixantrone maleate, Plakotenin, Platencin, Platensimycin, Plevitrexed, Pluraflavin E, Plusbacin A1, Plusbacin A2, Plusbacin A3, Plusbacin A4, Plusbacin B1, Plusbacin B2, Plusbacin B3, Plusbacin B4, Polyalthidin, Pomisartan, Ponalrestat, Poststatin, PPI17-24, Pradimicin A, Pradimicin B, Pradimicin D, Pradimicin E, Pradimicin FA-1, Pradimicin FA-2, Pradimicin FL, Pradimicin FS ((+)-enantiomer), Pradimicin L, Pradimicin Q, Pradimicin S, Pradimicin T1, Pradimicin T2, Pradofloxacin, Pralatrexate, Pranoprofen, Prefolic A, Pregabalin, Premafloxacin, Premafloxacin hydrochloride, Prezatide copper acetate, Proamipide, Probenecid, Probestin, Procysteine, Proglumide, Propagermanium, Propofol hemisuccinate, Prostatin, Prostratin succinate, Protocatechuic acid, Protoporphyrin IX gallium(III) complex, Prulifloxacin, Prulifloxacin Hydrochloride, Prulifloxacin Mesylate, Pseudomycin A', Pseudomycin B', Pycnanthuquinone A, Pycnanthuquinone B, Pyloricidin B, Pyridazomycin, Pyrrolosporin A, Quiflapon Sodium, Quinapril hydrochloride, Quinlukast, Rafabegron, Ragaglitazar, Raltitrexed, Ramatroban, Ramipril, Raxofelast, Razupenem, Rebamipide bismuth citrate tetramethyledamine, Rebamipide bismuth L-tartrate tetramethyledamine, Repaglinide, Resobene, Reveromycin A, Rhododaurichromanic acid A, Ridogrel, Robenacoxib, Rocagloic acid, Rolafagrel, Romazarit, Romurtide, Rosaprostol sodium, Rosuvastatin calcium, Rosuvastatin sodium, Rufloxacin Gluconate, Rufloxacin hydrochloride, Rumycin 1, Rumycin 2, Salazopyridazin, Salcaprozic acid sodium salt, Salicylazobenzoic acid, S-Allylmercaptocaptopril, Salmisteine, Salvianolic acid L, Samixogrel, Sampatrilat, Sanfetrinem, Sanfetrinem sodium, Sapurimycin, Sarpogrelate hydrochloride, Saussureamine A, Saussureamine B, Saussureamine C, Saussureamine D, Saussureamine E, Scabronine G, Scopadulcic acid B, Securioside A, Securioside B, Selank, Semduramicin, Seocalcitol, Seratrodast, Serofendic acid, Sessiloside, Shepherdin, Sialosylcholesterol-Alpha Sodium Salt, Sitafloxacin hydrate, S-Nitrosocaptopril, S-Nitrosoglutathione, Sodelglitazar, Sodium cromoglycate, Sodium oxybate, Sofalcone, Solabegron hydrochloride, Sorbicillactone A, Sparfloxacin, Sphingofungin F, Spinorphin, Spirapril, Spiriprostil, Spiroglumide, Spiroximicin, Squalestatin I, Stachybocin A, Stachybocin B, Stachybocin C, Staplabin, Starrhizin, Sterenin D, Subtilopentadecanoic acid, Succinobucol, Sufotidine bismuth citrate, Sugammadex sodium, Sulfasalazine, Sulindac, Sulopenem, Sulukast, Sunflower trypsin inhibitor-1, Susalimod, Tafamidis meglumine, Tageflar, Talaglumetad hydrochloride, Talibegron, Talibegron hydrochloride, Talopterin, Taltobulin, Tamibarotene, Tanogitran, Tanomastat, TAP-doxorubicin, Tarenflurbil, Targinine, Tazarotenic Acid, Tebipenem, Teicoplanin-A2-1, Teicoplanin-A2-2, Teicoplanin-A2-3, Teicoplanin-A2-5, Telavancin hydrochloride, Telmesteine, Telmisartan, Temafloxacin hydrochloride, Temocapril hydrochloride, Temurtide, Tenosal, Terbogrel, Terestigmine tartrate, Terikalant fumarate, Tesaglitazar, Tetomilast, Tetradecylselenoacetic acid, Tetrafibricin, Tetragalloylquinic acid, Tetrahydroechinocandin B, Tetronothiodin, Tezampanel, Thermozymocidin, Thiazohalostatin, Thielavin G, Thielocin, Thielocin B3, Thiofoscarnet, Thioxamycin, Thrazarine, Thymic humoral factor gamma-2, Thymopentin, Tiagabine hydrochloride, Tibenelast, Ticolubant, Tilarginine hydrochloride, Tiliquinatine, Timodepressin, Tipelukast, Tiplasinin, Tirofiban hydrochloride, Tisartan, Tolfenamic acid, Tolmetin, Tolrestatin, Tomopenem, Tosufloxacin, Tosufloxacin Tosilate, Trandolapril, Trandolaprilat, Tranexamic acid, Tranilast, Treprostinil diethanolamine, Treprostinil, Tretinoin, Triacetylshikimic acid, Trichomycin A, Triflusal, Trimexautide, Trimoprostil, Tripterin, Tropesin, Trovafloxacin, Trovafloxacin hydrate, Trovafloxacin hydrochloride mesylate, Trovafloxacin mesilate, Tubelactomicin A, Tuberactomycin D, Tuberactomycin E, Tubulysin A, Tubulysin B, Tubulysin C, Tucaresol, Tuftsin, Turbinaric acid, Tyroservatide, Ubenimex, Ulifloxacin, Uncarinic acid A, Uncarinic acid B, Unoprostone, Ursodeoxycholic acid, Ursolic acid phosphate, Utibapril, Utibaprilat, Vadimezan, Valonomycin A, Valproate Semisodium, Valproic acid, Valsartan, Vancomycin hydrochloride, Varespladib, Vebufloxacin, Vedaprofen, Veliflapon, Verlukast, Vinaxanthone, Viquidacin, Viranamycin-A, Viscosin, Vitilevuamide, Voreloxin, W Peptide, Xanthofulvin, Zabicipril Hydrochloride, Zabiciprilat Hydrochloride, Zabofloxacin hydrochloride, Zaltoprofen, Zanamivir, Zaragozic acid D3, Zenarestat, Zofenoprilat, Zofenoprilat arginine, Zolasartan, and Zonampanel.

Another aspect of the present invention is a pharmaceutical composition comprising a carrier-linked prodrug of the present invention, or a pharmaceutically acceptable salt thereof, and optionally one or more excipients.

The pharmaceutical compositions of the present invention are further described in the following paragraphs.

The pharmaceutical composition comprising the carrier-linked prodrug of the present invention may be provided as a liquid composition or as a dry composition. Suitable methods of drying are, for example, spray-drying and lyophilization (freeze-drying). A preferred method of drying is lyophilization.

Preferably, the carrier-linked prodrug is sufficiently dosed in the composition to provide a therapeutically effective amount of the biologically active moiety for at least one day in one application in the case of therapeutically active moieties. More preferably, one application of the pharmaceutical composition comprising the carrier-linked prodrug is sufficient for at least 12 hours, such as for one day, two days, such as three days, four days, five days, six days, or is sufficiently dosed for at least one week, such as for one week, two weeks, three weeks, four weeks, five weeks, six weeks, seven weeks, eight weeks, three months, four months, five months, six months or one year.

In one embodiment, the pharmaceutical composition comprises more than one carrier-linked prodrug of the present invention. Said one or more carrier-linked prodrugs may comprise different reversible prodrug linker moieties having different or the same half-lives, may comprise different biologically active moieties, and/or may comprise different carrier moieties.

In another embodiment the pharmaceutical composition further comprises other biologically active moieties in their free form or as prodrugs, i.e. as prodrugs other than those of the present invention.

The pharmaceutical composition of carrier-linked prodrug according to the present invention optionally comprises one or more excipients.

Excipients may be categorized as buffering agents, isotonicity modifiers, preservatives, stabilizers, anti-adsorption agents, oxidation protection agents, viscosifiers/viscosity enhancing agents, or other auxiliary agents. In some cases, these ingredients may have dual or triple functions. The pharmaceutical compositions of carrier-linked prodrugs according to the present invention contain one or more excipients, selected from the groups consisting of:
(i) Buffering agents: physiologically tolerated buffers to maintain pH in a desired range, such as sodium phosphate, bicarbonate, succinate, histidine, citrate and acetate, sulphate, nitrate, chloride, pyruvate. Antacids such as Mg(OH)₂ or ZnCO₃ may be also used. Buffering capacity may be adjusted to match the conditions most sensitive to pH stability;
(ii) Isotonicity modifiers: to minimize pain that can result from cell damage due to osmotic pressure differences at the injection depot. Glycerin and sodium chloride are examples. Effective concentrations can be determined by osmometry using an assumed osmolality of 285-315 mOsmol/kg for serum;
(iii) Preservatives and/or antimicrobials: multidose parenteral preparations require the addition of preservatives at a sufficient concentration to minimize risk of patients becoming infected upon injection and corresponding regulatory requirements have been established. Typical preservatives include m-cresol, phenol, methylparaben, ethylparaben, propylparaben, butylparaben, chlorobutanol, benzyl alcohol, phenylmercuric nitrate, thimerosol, sorbic acid, potassium sorbate, benzoic acid, chlorocresol, and benzalkonium chloride;
(iv) Stabilizers: Stabilization is achieved by strengthening of the protein-stabilizing forces, by destabilization of the denatured state, or by direct binding of excipients to the protein. Stabilizers may be amino acids such as alanine, arginine, aspartic acid, glycine, histidine, lysine, proline, sugars such as glucose, sucrose, trehalose, polyols such as glycerol, mannitol, sorbitol, salts such as potassium phosphate, sodium sulphate, chelating agents such as EDTA, hexaphosphate, ligands such as divalent metal ions (zinc, calcium, etc.), other salts or organic molecules such as phenolic derivatives. In addition, oligomers or polymers such as cyclodextrins, dextran, dendrimers, PEG or PVP or protamine or HSA may be used;
(v) Anti-adsorption agents: Mainly ionic or non-ionic surfactants or other proteins or soluble polymers are used to coat or adsorb competitively to the inner surface of the composition's or composition's container. Suitable surfactants are e.g., alkyl sulfates, such as ammonium lauryl sulfate and sodium lauryl sulfate; alkyl ether sulfates, such as sodium laureth sulfate and sodium myreth sulfate; sulfonates such as dioctyl sodium sulfosuccinates, perfluorooctanesulfonates, perfluorobutanesulfonates, alkyl benzene sulfonates; phosphates, such as alkyl aryl ether phosphates and alkyl ether phosphates; carboxylates, such as fatty acid salts (soaps) or sodium stearate, sodium lauroyl sarcosinate, perfluorononanoate, perfluorooctanoate; octenidine dihydrochloride; quaternary ammonium cations such as cetyl trimethylammonium bromide, cetyl trimethylammonium chloride, cetylpyridinium chloride, polyethoxylated tallow amine, benzalkonium chloride, benzethonium chloride, 5-bromo-5-nitor-1,3-dioxane, dimethyldioctadecylammonium chloride, dioctadecyldimethylammonium bromide; zwitterionics, such as 3-[(3-cholamidopropyl)dimethylammonio]-1-propanesulfonate, cocamidopropyl hydroxysultaine, amino acids, imino acids, cocamidopropyl betaine, lecithin; fatty alcohols, such as cetyl alcohol, stearyl alcohol, cetostearyl alcohol, oleyl alcohol; polyoxyethylene glycol alkyl ethers, such as octaethylene glycol monododecyl ether, pentaethylene glycol monododecyl ether; polyoxypropylene glycol alkyl ethers; glucoside alkyl ethers, such as decyl glucoside, lauryl glucoside, octyl glucoside; polyoxyethylene glycol octylphenol ethers such as Triton X-100; polyoxyethylene glycol alkylphenol ethers such as nonoxynol-9; glycerol alkyl esters such as glyceryl laurate; polyoxyethylene glycol sorbitan alkyl esters such as polysorbates; sorbitan alkyl esters; cocamide MEA and cocamide DEA; dodecyl dimethylamine oxide; block copolymers of polyethylene glycol and polypropylene glycol, such as poloxamers (Pluronic F-68), PEG dodecyl ether (Brij 35), polysorbate 20 and 80; other anti-absorption agents are dextran, polyethylene glycol, PEG-polyhistidine, BSA and HSA and gelatines. Chosen concentration and type of excipient depends on the effect to be avoided but typically a monolayer of surfactant is formed at the interface just above the CMC value;
(vi) Lyo- and/or cryoprotectants: During freeze- or spray drying, excipients may counteract the destabilizing effects caused by hydrogen bond breaking and water removal. For this purpose sugars and polyols may be used but corresponding positive effects have also been observed for surfactants, amino acids, non-aqueous solvents, and other peptides. Trehalose is particulary efficient at reducing moisture-induced aggregation and also improves thermal stability potentially caused by exposure of protein hydrophobic groups to water. Mannitol and sucrose may also be used, either as sole lyo/cryoprotectant or in combination with each other where higher ratios of mannitol:sucrose are known to enhance physical stability of a lyophilized cake. Mannitol may also be combined with trehalose. Trehalose may also be combined with sorbitol or sorbitol used as the sole protectant. Starch or starch derivatives may also be used;
(vii) Oxidation protection agents: antioxidants such as ascorbic acid, ectoine, methionine, glutathione, monothioglycerol, morin, polyethylenimine (PEI), propyl gallate, vitamin E, chelating agents such aus citric acid, EDTA, hexaphosphate, thioglycolic acid;
(viii) Spreading or diffusing agent: modifies the permeability of connective tissue through the hydrolysis of components of the extracellular matrix in the intrastitial space such as but not limited to hyaluronic acid, a polysaccharide found in the intercellular space of connective tissue. A spreading agent such as but not limited to hyaluronidase temporarily decreases the viscosity of the extracellular matrix and promotes diffusion of injected drugs;
(ix) Other auxiliary agents: such as wetting agents, viscosity modifiers, antibiotics, hyaluronidase. Acids and bases such as hydrochloric acid and sodium hydroxide are auxiliary agents necessary for pH adjustment during manufacture;

In one embodiment the pharmaceutical composition comprising the carrier-linked prodrugs of the present invention in either dry or liquid form may be provided as a single or multiple dose composition.

In one embodiment of the present invention, the liquid or dry pharmaceutical composition comprising the carrier-linked prodrug is provided as a single dose, meaning that the container in which it is supplied contains one pharmaceutical dose.

Alternatively, the liquid or dry pharmaceutical composition comprising the carrier-linked prodrug is a multiple dose composition, meaning that the container in which it is supplied contains more than one therapeutic dose, i.e., a multiple dose composition contains at least 2 doses. Such multiple dose composition of carrier-linked prodrug can either be used for different patients in need thereof or can be used for one patient, wherein the remaining doses are stored after the application of the first dose until needed.

In another aspect of the present invention the pharmaceutical composition is in a container. Suitable containers for liquid or dry compositions are, for example, syringes, vials, vials with stopper and seal, ampouls, and cartridges. In particular, the liquid or dry composition comprising the carrier-linked prodrug according to the present invention is provided in a syringe. If the pharmaceutical composition comprising the carrier-linked prodrug is a dry pharmaceutical composition the container preferably is a dual-chamber syringe. In such embodiment, said dry pharmaceutical composition is provided in a first chamber of the dual-chamber syringe and reconstitution solution is provided in the second chamber of the dual-chamber syringe.

Prior to applying the dry composition of carrier-linked prodrug to a patient in need thereof, the dry composition is reconstituted. Reconstitution can take place in the container in which the dry composition of carrier-linked prodrug is provided, such as in a vial, syringe, dual-chamber syringe, ampoule, and cartridge. Reconstitution is done by adding a predefined amount of reconstitution solution to the dry composition. Reconstitution solutions are sterile liquids, such as water or buffer, which may contain further additives, such as preservatives and/or antimicrobials, such as, for example, benzylalcohol and cresol. Preferably, the reconstitution solution is sterile water. When a dry composition is reconstituted, it is referred to as a "reconstituted pharmaceutical composition" or "reconstituted composition".

An additional aspect of the present invention relates to the method of administration of a reconstituted or liquid pharmaceutical composition comprising the carrier-linked prodrug of the present invention. The pharmaceutical composition comprising carrier-linked prodrug may be administered by methods of inhalation, injection or infusion, including intradermal, subcutaneous, intramuscular, intravenous, intraosseous, and intraperitoneal. Preferably, the pharmaceutical composition comprising carrier-linked prodrug is administered subcutaneously.

The preferred method of administration for dry pharmaceutical compositions comprising the carrier-linked prodrugs of the present invention is via subcutaneous injection.

Therefore, in a preferred embodiment, the present invention relates to a carrier-linked prodrug or a pharmaceutically acceptable salt thereof of the present invention or a pharmaceutical composition of the present invention, for use as medicament for topical, enteral administration, parenteral administration, inhalation, injection, or infusion, intraarticular, intradermal, subcutaneous, intramuscular, intravenous, intraosseous, and intraperitoneal, intrathecal, intracapsular, intraorbital, intracardiac, transtracheal, subcuticular, intraarticular, subcapsular, subarachnoid, intraspinal, intraventricular or intrasternal administration, preferably for subcutaneous administration.

Therefore, in another preferred embodiment, the present invention relates to a carrier-linked prodrug or a pharmaceutically acceptable salt thereof of the present invention or a pharmaceutical composition of the present invention, wherein such carrier-linked prodrug or pharmaceutically acceptable salt thereof or pharmaceutical composition is suitable to be administered to a patient via topical, enteral or parenteral administration and by methods of external application, inhalation, injection or infusion, including intraarticular, intradermal, subcutaneous, intramuscular, intravenous, intraosseous, and intraperitoneal, intrathecal, intracapsular, intraorbital, intracardiac, transtracheal, subcuticular, intraarticular, subcapsular, subarachnoid, intraspinal, intraventricular and intrasternal application, preferably by subcutaneous application.

A further aspect is a method of preparing a reconstituted composition comprising a diagnostically and/or therapeutically effective amount of carrier-linked prodrug of the present invention, and optionally one or more pharmaceutically acceptable excipients, the method comprising the step of
- contacting the pharmaceutical composition comprising carrier-linked prodrug of the present invention with a reconstitution solution.

Another aspect is a reconstituted pharmaceutical composition comprising a diagnostically and/or therapeutically effective amount of the carrier-linked prodrug of the present invention, and optionally one or more pharmaceutically acceptable excipients.

Another aspect of the present invention is the method of manufacturing a dry composition of carrier-linked prodrug. In one embodiment, such dry composition is made by
(i) admixing the carrier-linked prodrug with optionally one or more excipients,
(ii) transfering amounts equivalent to single or multiple doses into a suitable container,
(iii) drying the composition in said container, and
(iv) sealing the container.

Suitable containers are vials, syringes, dual-chamber syringes, ampoules, and cartridges.

Another aspect of the present invention is a kit of parts.

If the administration device is simply a hypodermic syringe then the kit may comprise the syringe, a needle and a container comprising the dry pharmaceutical composition of carrier-linked prodrug for use with the syringe and a second container comprising the reconstitution solution.

If the pharmaceutical composition is a liquid composition then the kit may comprise the syringe, a needle and a container comprising the liquid composition of carrier-linked prodrug for use with the syringe.

In more preferred embodiments, the injection device is other than a simple hypodermic syringe and so the separate container with reconstituted or liquid carrier-linked prodrug is adapted to engage with the injection device such that in use the liquid composition in the container is in fluid connection with the outlet of the injection device. Examples of administration devices include but are not limited to hypodermic syringes and pen injector devices. Particularly preferred injection devices are the pen injectors in which case the container is a cartridge, preferably a disposable cartridge. Optionally, the kit of parts comprises a safety device for the needle which can be used to cap or cover the needle after use to prevent injury.

A preferred kit of parts comprises a needle and a container containing the composition according to the present invention and optionally further containing a reconstitution solution, the container being adapted for use with the needle. Preferably, the container is a dual-chamber syringe.

The carrier-linked prodrugs of the present invention may be synthesized in different ways and it is understood that the method of synthesis depends on the exact structure of such carrier-linked prodrugs.

Another subject of the present invention is a method for the synthesis of a carrier-linked prodrug or a pharmaceutically acceptable salt thereof of the present invention. Carrier-linked prodrugs or precursors of such prodrugs according to the present invention may be prepared by known methods or in accordance with the reaction sequences described below. The starting materials used in the preparation (synthesis) of carrier-linked prodrugs of the invention or precursors thereof are known or commercially available, or can be prepared by known methods or as described below.

All reactions for the synthesis of the carrier-linked prodrugs according to the present invention including precursors are per se well-known to the skilled person and can be carried out under standard conditions according to or analogously to procedures described in the standard literature of organic chemistry. Depending on the circumstances of the individual case, in order to avoid side reactions during the synthesis of a carrier-linked prodrug or a precursor thereof, it can be necessary or advantageous to temporarily block functional groups by introducing protective groups and to deprotect them in a later stage of the synthesis, or introduce functional groups in the form of precursor groups which in a later reaction step are converted into the desired functional groups. Such synthesis strategies and protective groups and precursor groups which are suitable in an individual case are known to the skilled person. If desired, the carrier-linked prodrugs or precursors thereof can be purified by customary purification procedures, for example by recrystallization or chromatography.

In one embodiment, the carrier-linked prodrugs according to the present invention (or a pharmaceutically acceptable salt thereof) may be prepared by a method comprising the steps of converting the carboxylic acid of the biologically active moiety to a biologically active moiety reagent D-Y, wherein Y is a leaving group, and subsequently reacting the reagent D-Y with a hydroxyl-group containing reversible prodrug linker reagent L-OH, thus generating a biologically active moiety-reversible prodrug linker conjugate D-L by forming a carboxylic ester linkage. Afterwards, D-L may be bound to a carrier moiety POL to obtain the carrier-linked prodrug of a biologically active moiety comprising a carboxylic acid group according to the present invention. Alternatively, the carrier moiety POL may already be bound to L-OH.

It is understood that functional groups of D not involved in the synthesis of the carrier-linked prodrugs of the present invention may be protected with suitable protecting groups known to the person skilled in the art.

Y is a leaving group. Suitable leaving groups are known to a person skilled in the art. Preferably, if attached to D, Y is chloride, bromide, fluoride, nitrophenoxy, imidazolyl, N-hydroxysuccinimidyl, N-hydroxybenzotriazolyl, N-hydroxyazobenzotriazolyl, pentafluorophenoxy, 2-thiooxo-thiazolidinyl, or N-hydroxysulfosuccinimidyl.

The carrier-linked prodrug of the present invention can be prepared starting from a polymer by convenient methods known in the art. It is clear to a practitioner in the art that several routes exist. For example, a reversible prodrug linker covalently attached to the biologically active moiety D can be reacted with the reactive functional groups of the polymer of the carrier moiety POL. Alternatively, a polymer-reversible prodrug linker reagent may be prepared for subsequent reaction with a preferentially activated biologically active acid D-Y.

In case the polymer carrier comprises, preferably consists of a hydrogel, it is preferred that the hydrogel is generated through chemical ligation reactions. The hydrogel may be formed from two macromolecular educts with complementary functionalities which undergo a reaction such as a condensation or addition. One of these starting materials is a crosslinker reagent with at least two identical functional groups and the other starting material is a homomultifunctional backbone reagent. Suitable functional groups present on the crosslinker reagent include terminal amino, carboxylic acid and derivatives, maleimide and other alpha,beta unsaturated Michael acceptors like vinylsulfone, thiol, hydroxyl groups. Suitable functional groups present in the backbone reagent include but are not limited to amino, carboxylic acid and derivatives, maleimide and other alpha,beta unsaturated Michael acceptors like vinylsulfone, thiol, hydroxyl groups. If the crosslinker reagent reactive functional groups are used substoichiometrically with respect to backbone reactive functional groups, the resulting hydrogel will be a reactive hydrogel with free reactive functional groups attached to the backbone structure.

If the carrier moiety POL is a hydrogel, a preferred method of synthesis is disclosed in WO-A 2011/042450, which is incorporated herein by reference, with the proviso, that instead of paliperidone, a carboxyl-comprising biologically active moiety is used and in which the functional group of the hydrogel is a hydroxyl group.

In another aspect, the invention provides a cartridge comprising a pharmaceutical composition of carrier-linked prodrug as hereinbefore described for use with a pen injector device. The cartridge may contain a single dose or multiplicity of doses of the carrier-linked prodrug.

Yet another aspect of the present invention is a carrier-linked prodrug of the present invention or pharmaceutically acceptable salt thereof, or a pharmaceutical composition of the present invention for use as a medicament.

In another embodiment, the present invention relates to the use of a carrier-linked prodrug of the present invention or a pharmaceutically acceptable salt thereof, or a pharmaceutical composition of the present invention for the preparation of a medicament and/or diagnostic.

It is understood, that the disease that can be treated and/or diagnosed a carrier-linked prodrug of the present invention or a pharmaceutically acceptable salt thereof, or a pharmaceutical composition of the present invention depends on the biologically active agent. A carrier-linked prodrug with an active agent moiety which has anti-cancer activity, like TAP-doxorubicin, is typically administered to a cancer patient. Analogously, a carrier-linked prodrug with an active agent moiety which has anti-inflammatory activity, like aminosalicylic acid, is typically administered to a patient who suffers from an inflammatory disease, like rheumatoid arthritis, IBD or Morbus Crohn. Analogously, a carrier-linked prodrug with an active agent moiety which has neurological activity is typically administered to a patient suffering from a neurological disease like Alzheimer's disease or Parkinson's disease. Analogously, a carrier-linked prodrug with an active agent moiety which has anti-infective activity, like Ciluprevir, is typically administered to a patient suffering from a infectious disease like bacterial, viral, protozoal or fungal infection.

In case the carrier-linked prodrugs according to the invention contain one or more acidic or basic groups, the invention also comprises their corresponding pharmaceutically or toxicologically acceptable salts, in particular their pharmaceutically acceptable salts. Thus, the carrier-linked prodrugs according to the invention which contain acidic groups can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Carrier-linked prodrugs according to the invention which contain one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the carrier-linked prodrugs according to the invention simultaneously contain acidic and basic groups in the molecule, the invention also includes, in addition to the salt forms mentioned, inner salts or betaines (zwitterions). The respective salts can be obtained by customary methods which are known to the person skilled in the art like, for example by contacting these with an organic or inorganic acid or base in a solvent or dispersant, or by anion exchange or cation exchange with other salts. The present invention also includes all salts of the prodrugs which, owing to low physiological compatibility, are not directly suitable for use in pharmaceuticals but which can be used, for example, as intermediates for chemical reactions or for the preparation of pharmaceutically acceptable salts.

Yet another aspect of the present invention is a method of diagnosing, treating, controlling, delaying or preventing in a mammalian patient, preferably in a human, in need of the treatment of one or more conditions comprising administering to said patient a diagnostically and/or therapeutically effective amount of a carrier-linked prodrug of the present invention or a pharmaceutically acceptable salt thereof or a pharmaceutical composition of the present invention.

### Methods and Analytics:

### Automated Flash Chromatography

Automated Flash Chromatography was performed on a Biotage "Isolera one" purification system Biotage AB, Sweden, using Biotage KP-Sil silica cartridges. Products were detected and collected at 254 and 280nm.

### Analytical RP-HPLC

Analytical RP-HPLC/ESI-MS was performed on waters equipment consisting of a 2695 sample manager, a 2487 Dual Absorbance Detector, and a ZQ 4000 ESI instrument equipped with a 5 µm Reprosil Pur 300 Å ODS-3 column (75 x 1.5 mm) (Dr. Maisch, Ammerbuch, Germany; flow rate: 350 µl/min, typical gradient: 10-90% MeCN in water, 0.05 % TFA over 5 min).

RP-UPLC/ESI-MS was performed on Waters/Thermo equipment consisting of a Waters Acquity UPLC with an Acquity PDA detector coupled to a Thermo LTQ Orbitrap Discovery high resolution/high accuracy mass spectrometer equipped with a ACQUITY UPLC® BEH300 C18 RP column (Waters Corporation, 2.1 x 50 mm, 300 Å, 1.7 µm, Flow: 0.25 mL/min; solvent A: UP-H₂0 + 0.04% TFA, solvent B: UP-MeCN + 0.05 % TFA.

Typical gradients for determination of released treprostinil from TransCon 5 kDa PEG linker treprostinil are: 0.25 mL flow rate, gradient: 30-50 % B over 10 min

### RP-HPLC purification:

For preparative RP-HPLC a Waters 600 controller and a 2487 Dual Absorbance Detector was used equipped with the following column: XBridge™ BEH300 Prep C18 5 µm, 10 x 150 mm column.

Typical gradients for purification procedures are:
- 6 mL/min flow rate, solvent A: H₂0 + 0.05% TFA, solvent B: MeCN + 0.05 % TFA, typical gradient: 1-95 % B over 14 min
- 6 mL/min flow rate, solvent A: H₂0 + 0.05% TFA, solvent B: MeCN + 0.05 % TFA, typical gradient: 10-80 % B over 14 min

HPLC fractions containing product were lyophilized.

### Chemicals and drug substances:

Treprostinil acid was purchased from Shanghai Techwell Biopharmaceutical Co., Ltd. or Chirogate International Inc.. All other chemicals were purchased from Sigma Aldrich GmbH.

Water and acetonitrile for analytical RP-HPLC were purchased from Biosolve B.V. and TFA from Thermo scientific.

### Example 1:

### Benzyl protection of 3-hydroxybutanoic acid 1:

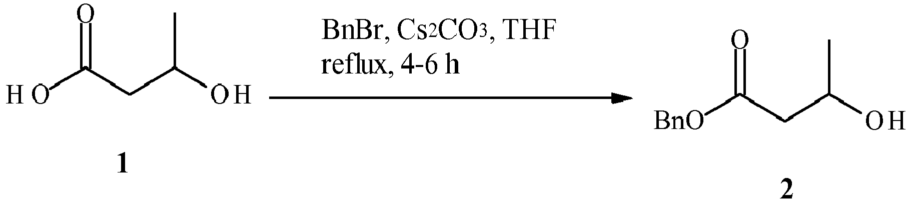

3-Hydroxybutanoic acid **1** (434 mg, 4.17 mmol) was dissolved in THF (10 mL) and BnBr (700 µL, 5.89 mmol) and Cs₂CO₃ (2.5 g, 7.67 mmol) were added. The reaction mixture was refluxed in a sealed tube for 4-6 hours. After cooling down to room temperature the reaction mixture was filtrated and the residue was washed several times with EtOAc. The organic solvents were removed and the product was purified by automated flash chromatography on silica in one portion (SNAP 25 g cartridge, flow 30 ml/min, solvent A: CH₂Cl₂, solvent B: MeOH; gradient: 0 - 5 % B over 19 CV) to remove starting material and obtain desired benzyl protected 3-hydroxybutanoic acid **2** as yellow oil.
Yield: 361 mg (45 %)
MS: m/z 217.1 = [M+Na]⁺ (MW+Na calculated = 217.2).

### Example 2:

### Coupling reaction of benzylated 3-hydroxybutanoic acid 2 with treprostinil:

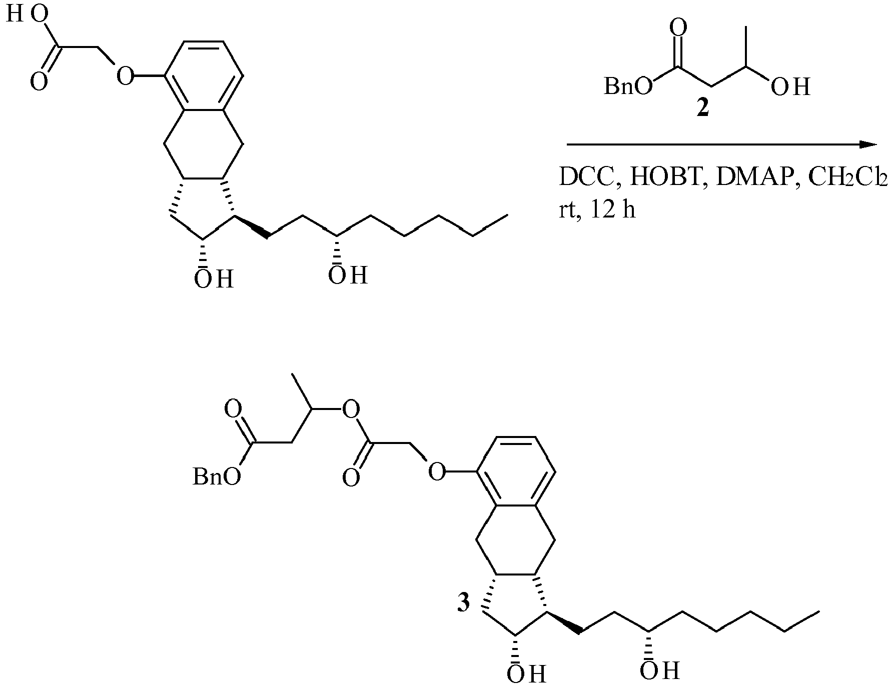

Treprostinil acid (10.5 mg, 0.0268 mmol) was dissolved in CH₂Cl₂ (4.5 mL) and DCC (9.4 mg, 0.0455 mmol), HOBT (7.5 mg, 0.0489 mmol) and DMAP (7.5 mg, 0.0613 mmol) were added to the solution. Then benzylated 3-hydroxybutanoic acid **2** (15 mg, 0.0772 mmol) was dissolved in CH₂Cl₂ (0.5 mL) and added to the reaction mixture. The mixture was stirred at RT until the consumption was complete (analytical RP-HPLC). Volatile solvents were removed *in vacuo* and the residue was purified over a small silica column (3 ml silica, DCM / MeOH (100 : 0) - DCM / MeOH (95 : 5) to obtain the desired linker treprostinil 3 as yellow oil.
Yield: 8 mg (50 %)
MS: m/z 589.3 = [M+Na]⁺ (MW+Na calculated = 589.7)

### Example 3:

### Hydrogenation reaction of benzylester 3:

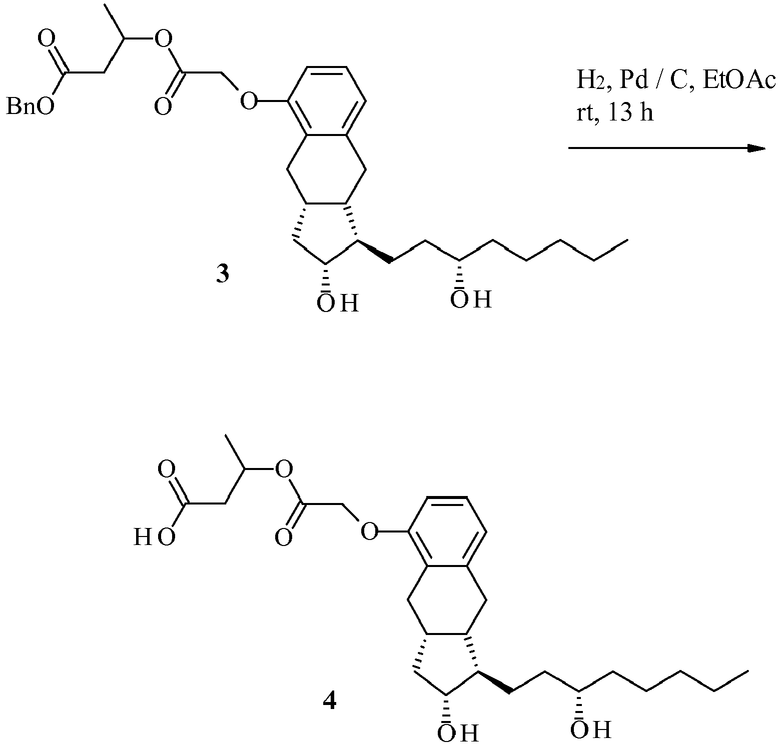

Benzylester **3** (13 mg, 0.0229 mmol) was dissolved in EtOAc (4 Å MS, 2 mL) and 5 % palladium on charcoal (5 % Pd, 15 mg) was added. Hydrogen was bubbled through the solution for 30 min. The reaction mixture was stirred further 12.5 h under hydrogen atmosphere until the consumption was complete (analytical RP-HPLC). The mixture was filtered over celite and washed several times with EtOAc. Organic solvents were removed *in vacuo* and the residue was purified using RP-HPLC (solvent A: H₂O with 0.05 % TFA, solvent B: MeCN with 0.05 % TFA, gradient: 1-95 % B over 20 min, flow: 6 mL/min). The product containing fractions were pooled and lyophilized to obtain **4** as white solid.
Yield: 1.9 mg (29 %).
MS: m/z 499.3 = [M+Na]⁺ (MW+Na calculated = 499.6).

### Example 4:

### Coupling reaction of 5 kDa PEG amine with linker treprostinil 4:

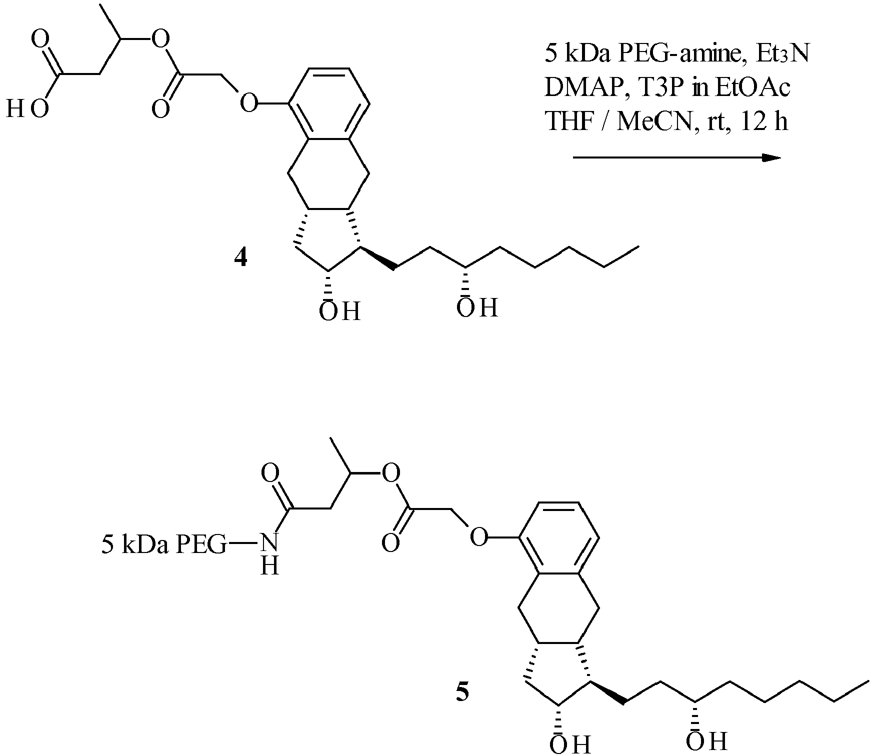

Linker treprostinil **4** (1.9 mg, 3.98 µmol) and 5 kDa PEG-amine (86 mg, 17.2 µmol) were dissolved in THF/MeCN (4 Å MS; 1.5 mL : 0.5 mL) and Et₃N (40 µL), a catalytic amount of DMAP and T3P (50% in EtOAc, 50 µL, 73.2 µmol) were successively added. The reaction mixture was allowed to stir at rt for 12 h. The reaction mixture was diluted with 20 µL H₂O and volatile solvents were removed *in vacuo.* The residue was purified using RP-HPLC (solvent A: H₂O with 0.05 % TFA, solvent B: MeCN with 0.05 % TFA, gradient: 10-80 % B over 20 min, flow: 6 mL/min). The product containing fractions were pooled and lyophilized to obtain TransCon PEG linker treprostinil **5** as white solid.
Yield: 12.5 mg (58 %).
MS: m/z 1378.6 = [M+4H]⁴⁺ (calculated = 1378.9) for one representative peak in the polymer distribution.

### Example 5:

### Treprostinil release kinetics of TransCon PEG linker treprostinil 5:

TransCon PEG linker treprostinil **5** (0.5 - 1.5 mg) was incubated in pH 7.4 hydrolysis buffer (60 mM sodium phosphate, 3 mM EDTA, 0.05 % Tween-20, 1 mL) at 37 °C and aliquots were analyzed by UPLC at various time points for released treprostinil.

### Half life determination of hydrolysis kinetics of TransCon PEG linker treprostinil 5:

The percentage of released treprostinil after incubation at pH 7.4 and 37°C for a given time period was determined by integrating the corresponding peaks in the RP-UPLC chromatogram. The relative percentage of free treprostinil at each time point was calculated from the free treprostinil peak area divided by the total sum of the peak areas corresponding to TransCon PEG linker treprostinil and treprostinil multiplied by 100. The data as shown in table 1 were subsequently plotted against time using an exponential fit assuming first order kinetics obtaining a half life of 4.20 d for the release kinetics of 5 at 37 °C and pH 7.4.

**Table 1:**

| **entry** | **Incubation time [d]** | **released treprostinil [%]** |
|---|---|---|
| 1 | 0.000 | 2 |
| 2 | 0.83 | 5 |
| 3 | 1.11 | 18 |
| 4 | 1.81 | 27 |
| 5 | 2.06 | 29 |
| 6 | 5.13 | 59 |
| 7 | 6.10 | 64 |
| 8 | 8.80 | 77 |
| 9 | 11.90 | 86 |

### Abbreviations

- AcOH: acetic acid
- BnBr: benzylbromide
- DMSO: dimethyl sulfoxide
- EDTA: Ethylenediamine tetraacetic acid disodium salt dihydrate
- EtOAc: Ethyl acetate
- eq: equivalent
- HPLC: High performance liquid chromatography
- MeOH: methanol
- MeCN: acetonitrile
- m/z: Mass/charge
- NaOH: Sodium hydroxide
- PEG: Polyethylene glycol
- RT: room temperature
- RP: Reversed phase
- T: temperature
- TransCon: Transient conjugated
- THF: tetrahydrofuran
- TFA: trifluoroacidic acid
- T3P: Propyl phosphonic anhydride
- UPLC: Ultra performance liquid chromatography
- UV: Ultra violet

## Claims

1. A carrier-linked prodrug of formula (Ia) or (Ib): 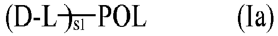
D(̵L-POL)ₛ₂ (Ib),
wherein
each D is individually a biologically active moiety comprising at least one carboxylic acid group;
each POL is individually a carrier moiety which comprises, preferably consists of a polymer with a molecular weight of from 5 kDa to 100 kDa,
s1 is an integer ranging from 1 to 64, preferably ranging from 1 to 16, even more preferably s1 is selected from 1, 2, 3, 4, 5, 6, 7 and 8,
s2 is an integer ranging from 1 to 16, preferably ranging from 1 to 8, even more preferably s2 is selected from 1, 2, 3, and 4,
each L is individually a reversible prodrug linker of formula (Ic): 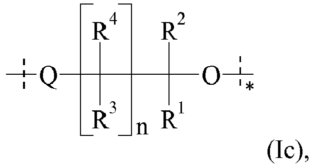
wherein the dashed line marked with an asterisk indicates attachment to the carboxyl group of a biologically active moiety D by forming a carboxylic ester comprising O and the other dashed line indicates attachment to the rest of the molecule;
R¹ is selected from the group of unsubstituted alkyl; substituted alkyl; unsubstituted phenyl; substituted phenyl; unsubstituted naphthyl; substituted naphthyl; unsubstituted indenyl; substituted indenyl; unsubstituted indanyl; substituted indanyl; unsubstituted tetralinyl; substituted tetralinyl; unsubstituted C₃₋₁₀ cycloalkyl; substituted C₃₋₁₀ cycloalkyl; unsubstituted 4- to 7-membered heterocyclyl; substituted 4- to 7-membered heterocyclyl; unsubstituted 9- to 11-membered heterobicyclyl; and substituted 9- to 11-membered heterobicyclyl;
R² is selected from H, unsubstituted alkyl, and substituted alkyl;
R³ and R⁴ are independently selected from the group consisting of H, unsubstituted alkyl, and substituted alkyl;
n is 0 or 1;
optionally, R¹ and R³ are joined together with the atoms to which they are attached to form a ring A;
A is selected from the group consisting of C₃₋₁₀ cycloalkyl; 4- to 7-membered aliphatic heterocyclyl; and 9- to 11-membered aliphatic heterobicyclyl, wherein A is unsubstituted or substituted;
Q is a spacer moiety;
or a pharmaceutically acceptable salt thereof.

2. The carrier-linked prodrug or a pharmaceutically acceptable salt thereof of claim 1, wherein the carrier-linked prodrug is of formula (Ia) with s1 being 1.

3. The carrier-linked prodrug or a pharmaceutically acceptable salt thereof of claim 1 or 2, wherein R² is H.

4. The carrier-linked prodrug or a pharmaceutically acceptable salt thereof of any of claims 1 to 3, wherein R¹ is selected from the group consisting of C₁₋₆ alkyl or substituted C₁₋₆ alkyl, more preferably C₁₋₄ alkyl or substituted C₁₋₄ alkyl, even more preferably, R¹ is selected from methyl, ethyl, n-propyl, isopropyl, n-butyl, isobutyl, sec-butyl, t-butyl, and benzyl.

5. The carrier-linked prodrug or a pharmaceutically acceptable salt thereof of any of claims 1 to 4, wherein R³ is selected from the group consisting of H, C₁₋₆ alkyl or substituted C₁₋₆ alkyl.

6. The carrier-linked prodrug or a pharmaceutically acceptable salt thereof of any of claims 1 to 5, wherein R⁴ is selected from the group consisting of H, C₁₋₆ alkyl or substituted C₁₋₆ alkyl.

7. The carrier-linked prodrug or a pharmaceutically acceptable salt thereof of any of claims 1 to 6, wherein POL of formula (Ia) and (Ib) comprises a polymer selected from the group consisting of polypeptides, 2-methacryloyl-oxyethyl phosphoyl cholins, hydrogels, poly(acrylic acids), poly(acrylates), poly(acrylamides), poly(alkyloxy) polymers, poly(amides), poly(amidoamines), poly(amino acids), poly(anhydrides), poly(aspartamides), poly(butyric acids), poly(glycolic acids), polybutylene terephthalates, poly(caprolactones), poly(carbonates), poly(cyanoacrylates), poly(dimethylacrylamides), poly(esters), poly(ethylenes), poly(ethyleneglycols), poly(ethylene oxides), poly(ethyl phosphates), poly(ethyloxazolines), poly(glycolic acids), poly(hydroxyethyl acrylates), poly(hydroxyethyloxazolines), poly(hydroxymethacrylates), poly(hydroxypropylmethacrylamides), poly(hydroxypropyl methacrylates), poly(hydroxypropyloxazolines), poly(iminocarbonates), poly(lactic acids), poly(lactic-co-glycolic acids), poly(methacrylamides), poly(methacrylates), poly(methyloxazolines), poly(organophosphazenes), poly(ortho esters), poly(oxazolines), poly(propylene glycols), poly(siloxanes), poly(urethanes), poly(vinyl alcohols), poly(vinyl amines), poly(vinylmethylethers), poly(vinylpyrrolidones), silicones, celluloses, carbomethyl celluloses, hydroxypropyl methylcelluloses, chitins, chitosans, dextrans, dextrins, gelatins, hyaluronic acids and derivatives, functionalized hyaluronic acids, mannans, pectins, rhamnogalacturonans, starches, hydroxyalkyl starches, hydroxyethyl starches and other carbohydrate-based polymers, xylans, and copolymers thereof.

8. The carrier-linked prodrug or a pharmaceutically acceptable salt thereof of any one of claims 1 to 7, wherein POL comprises, preferably consists of a water-soluble polymer.

9. The carrier-linked prodrug or a pharmaceutically acceptable salt thereof of any one of claims 1 to 8, wherein POL has a molecular weight from 5 to 80 kDa.

10. The carrier-linked prodrug or a pharmaceutically acceptable salt thereof of any one of claims 1 to 9, wherein POL has a molecular weight from 10 to 40 kDa.

11. The carrier-linked prodrug or a pharmaceutically acceptable salt thereof of any one of claims 1 to 7, wherein POL is water-insoluble.

12. The carrier-linked prodrug or a pharmaceutically acceptable salt thereof of any one of claims 1 to 7 or 11, wherein POL is a cross-linked hydrogel.

13. A pharmaceutical composition comprising a carrier-linked prodrug or a pharmaceutically acceptable salt thereof of any one of claims 1 to 12, and optionally one or more pharmaceutically acceptable excipients.

14. The carrier-linked prodrug or the pharmaceutically acceptable salt thereof of any one of claims 1 to 12 or the pharmaceutical composition of claim 13 for use as a medicament.

15. The carrier-linked prodrug or the pharmaceutically acceptable salt thereof of any one of claims 1 to 12 or the pharmaceutical composition of claim 13 for use as medicament for topical, enteral administration, parenteral administration, inhalation, injection, infusion, intraarticular, intradermal, subcutaneous, intramuscular, intravenous, intraosseous, and intraperitoneal, intrathecal, intracapsular, intraorbital, intracardiac, transtracheal, subcuticular, intraarticular, subcapsular, subarachnoid, intraspinal, intraventricular or intrasternal administration, preferably for subcutaneous administration.


SEQUENCE LISTING

<110>  Ascendis Pharma A/S

<120>  Carrier-linked prodrugs having reversible carboxylic ester
       linkages

<130>  CPX67835PCEPT1

<160>  15

<170>  PatentIn version 3.5

<210>  1
<211>  20
<212>  PRT
<213>  Artificial Sequence

<220>
<223>  polymer cassette

<400>  1

Ala Ala Ala Ala Ser Ser Ala Ser Ser Ala Ser Ser Ser Ser Ser Ala
1               5                   10                  15

Ala Ala Ser Ala
            20

<210>  2
<211>  20
<212>  PRT
<213>  Artificial Sequence

<220>
<223>  polymer cassette

<400>  2

Ala Ala Ser Ala Ala Ala Ser Ser Ala Ala Ala Ser Ala Ala Ala Ala
1               5                   10                  15

Ser Ala Ser Ser
            20

<210>  3
<211>  20
<212>  PRT
<213>  Artificial Sequence

<220>
<223>  polymer cassette

<400>  3

Ala Ser Ala Ser Ala Ser Ala Ser Ala Ser Ala Ser Ser Ala Ala Ser
1               5                   10                  15

Ala Ala Ser Ala
            20

<210>  4
<211>  20
<212>  PRT
<213>  Artificial Sequence

<220>
<223>  polymer cassette

<400>  4

Ser Ala Ala Ser Ser Ser Ala Ser Ser Ser Ser Ala Ala Ser Ser Ala
1               5                   10                  15

Ser Ala Ala Ala
            20

<210>  5
<211>  20
<212>  PRT
<213>  Artificial Sequence

<220>
<223>  polymer cassette

<400>  5

Ser Ser Ser Ser Ala Ala Ser Ala Ala Ser Ala Ala Ala Ala Ala Ser
1               5                   10                  15

Ser Ser Ala Ser
            20

<210>  6
<211>  20
<212>  PRT
<213>  Artificial Sequence

<220>
<223>  polymer cassette

<400>  6

Ser Ser Ala Ser Ser Ser Ala Ala Ser Ser Ser Ala Ser Ser Ser Ser
1               5                   10                  15

Ala Ser Ala Ala
            20

<210>  7
<211>  20
<212>  PRT
<213>  Artificial Sequence

<220>
<223>  polymer cassette

<400>  7

Ser Ala Ser Ala Ser Ala Ser Ala Ser Ala Ser Ala Ala Ser Ser Ala
1               5                   10                  15

Ser Ser Ala Ser
            20

<210>  8
<211>  20
<212>  PRT
<213>  Artificial Sequence

<220>
<223>  polymer cassette

<400>  8

Ala Ser Ser Ala Ala Ala Ser Ala Ala Ala Ala Ser Ser Ala Ala Ser
1               5                   10                  15

Ala Ser Ser Ser
            20

<210>  9
<211>  20
<212>  PRT
<213>  Artificial Sequence

<220>
<223>  polymer cassette

<400>  9

Ala Ser Pro Ala Ala Pro Ala Pro Ala Ser Pro Ala Ala Pro Ala Pro
1               5                   10                  15

Ser Ala Pro Ala
            20

<210>  10
<211>  20
<212>  PRT
<213>  Artificial Sequence

<220>
<223>  polymer cassette

<400>  10

Ala Ala Pro Ala Ser Pro Ala Pro Ala Ala Pro Ser Ala Pro Ala Pro
1               5                   10                  15

Ala Ala Pro Ser
            20

<210>  11
<211>  20
<212>  PRT
<213>  Artificial Sequence

<220>
<223>  polymer cassette

<400>  11

Ala Pro Ser Ser Pro Ser Pro Ser Ala Pro Ser Ser Pro Ser Pro Ala
1               5                   10                  15

Ser Pro Ser Ser
            20

<210>  12
<211>  20
<212>  PRT
<213>  Artificial Sequence

<220>
<223>  polymer cassette

<400>  12

Ser Ser Pro Ser Ala Pro Ser Pro Ser Ser Pro Ala Ser Pro Ser Pro
1               5                   10                  15

Ser Ser Pro Ala
            20

<210>  13
<211>  24
<212>  PRT
<213>  Artificial Sequence

<220>
<223>  polymer cassette

<400>  13

Ala Ala Ser Pro Ala Ala Pro Ser Ala Pro Pro Ala Ala Ala Ser Pro
1               5                   10                  15

Ala Ala Pro Ser Ala Pro Pro Ala
            20

<210>  14
<211>  20
<212>  PRT
<213>  Artificial Sequence

<220>
<223>  polymer cassette

<400>  14

Ala Ser Ala Ala Ala Pro Ala Ala Ala Ser Ala Ala Ala Ser Ala Pro
1               5                   10                  15

Ser Ala Ala Ala
            20

<210>  15
<211>  20
<212>  PRT
<213>  Artificial Sequence

<220>
<223>  polymer cassette

<400>  15

Ser Ala Pro Ser Ser Pro Ser Pro Ser Ala Pro Ser Ser Pro Ser Pro
1               5                   10                  15

Ala Ser Pro Ser
            20